# EUROPEAN PATENT APPLICATION

(11) **EP 4 006 160 A1**
(43) Date of publication of application: **01.06.2022**
(21) Application number: 19937398.6
(22) Date of filing: 12.07.2019
(51) Int. Cl.: C12N 15/864, A61K 48/00, A61P 43/00, C12N 7/01, C12N 15/35

(54) **ADENO-ASSOCIATED VIRUS VIRION FOR GENE TRANSFER TO HUMAN LIVER**

(71) Applicant: Gene Therapy Research Institution Co., Ltd., Kawasaki-ku Kawasaki-shi Kanagawa 2100821 (JP)
(72) Inventor: MURAMATSU, Shin-ichi, Shimotsuke-shi, Tochigi 329-0498 (JP); TAKINO, Naomi, Shimotsuke-shi, Tochigi 329-0498 (JP); ITO, Mika, Shimotsuke-shi, Tochigi 329-0498 (JP)
(74) Representative: Elkington and Fife LLP
(86) International application number: PCT/JP2019/027717
(87) International publication number: WO 2021/009805

(57) **Abstract**

The present application provides a modified adeno-associated virus (AAV) vector that efficiently transfers genes to a liver in a living body (human, for example) by reducing the attack from neutralizing antibodies in blood. More specifically, the present application provides an adeno-associated virus vector comprising a capsid protein having an amino acid sequence which has at least one of serine at position 472, serine at position 587, and asparagine at position 706 in the amino acid sequence represented by SEQ ID NO: 2 or 3 substituted with another amino acid, and has 1 to 6 amino acid residues at other residue positions deleted, substituted or inserted. Said adeno-associated virus vector does not cross-react with a neutralizing antibody against AAV serotype 2 (AAV2).

## Description

### TECHNICAL FIELD

The present invention relates to a recombinant adeno-associated virus (AAV) vector that is less susceptible to a neutralizing antibody in a serum. More particularly, the present invention relates to a mutant AAV having reduced cross-reactivity with a neutralizing antibody in a serum of a living body, allowing more efficient transfer of a gene into a liver of a living body (e.g., human).

### BACKGROUND ART

Gene transfer vectors adopting an adeno-associated virus (AAV) can transfer genes into cells including nerve cells, hepatocytes (hepatic parenchymal cells), retinal cells, muscle cells, myocardial cells, vascular endothelial cells and adipocytes *in vivo,* and allow expression of the genes for a prolonged period of time (Patent documents 1-3). For this reason, clinical application of such vectors for use as gene therapy vectors for hemophilia, retinitis pigmentosa, Parkinson's disease, etc. has been developed (Non-Patent Documents 1 and 2). In addition, such vectors have been frequently used as vectors for transferring genes of sgRNA and CAS9 protein in recent cases of gene editing (Patent document 3, and Non-patent document 3). For hemophilia, it has been reported that the gene therapy by transferring an AAV vector expressing factor VIII or factor IX into hepatocytes provides desired results (Patent document 4, and Non-patent documents 4, 5).

However, AAV3 and/or AAV8, which are highly efficient in transferring a gene into hepatocytes, are cross-reactive with a neutralizing antibody against AAV2 found in over 60% of adults, and thus it has been reported that the AAV3 and AAV8 cannot be expected to exert a sufficient effect in most patients (Non-patent document 6-9). In addition, there are ongoing studies for performing a gene therapy to patients who have not previously been targeted by the gene therapy because they possess the neutralizing antibody, for performing a gene therapy once more for patients who have not achieved satisfactory effects by the previous gene therapy, and the like.

### PRIOR ART DOCUMENTS

### Patent documents

Patent document 1: International Patent Application Publication WO2008/124724
Patent document 2: International Patent Application Publication WO2012/057363
Patent document 3: International Patent Application Publication WO2018/131551
Patent document 4: Japanese Patent Application National Publication No. 2016-525356

### Non-patent documents

Non-patent document 1: Dunber CE, et al., Science 359: eaan4672, 2018.
Non-patent document 2: Hastie E, Samulski RJ, Hum Gene Ther 26: 257-265, 2015.
Non-patent document 3: Ohmori T, et al., Sci Rep 7: 4159, 2017.
Non-patent document 4: George LA, et al., N Engl J Med 377: 2215-2227, 2017.
Non-patent document 5: Rangarajan S, et al., N Engl J Med 377: 2519-2530, 2017.
Non-patent document 6: Mimuro J, et al., J Med Virol 86: 1990-1997, 2014.
Non-patent document 7: Ling C, et al., J Integr Med 13: 341-346, 2015.
Non-patent document 8: Meliani A, et al., Hum Gene Ther Methods 26: 45-53, 2015
Non-patent document 9: Grieg JA, et al, Hum Gene Ther. 29: 1364-1375, 2018

### SUMMARY OF INVENTION

### Problems to be solved by the invention

Under such circumstances, an AAV vector, which is less cross-reactive with a neutralizing antibody against AAV2 or the like in the serum and is highly efficient in transferring a gene into hepatocytes, for example, a novel AAV vector derived from AAV3 or AAV8, has been desired.

### Means for solving the problems

In order to solve the above-described problem, the present inventors have gone through various trials and errors, and created a novel and unconventional modified AAV vector which is less cross-reactive with a neutralizing antibody in a serum, by modifying a portion of the amino acids of the coat protein (capsid) of AAV. Furthermore, the present inventors found that use of the modified vector further enhances the efficiency of gene transfer into cultured human liver-derived cells, thereby accomplishing the present invention.

Specifically, the present invention provides inventions exemplified below, including an AAV vector which is less cross-reactive with a neutralizing antibody in a serum and which provides highly efficient gene transfer into hepatocytes, for example, a recombinant AAV for a gene therapy targeting hepatocytes, a pharmaceutical composition comprising the same, and the like.
[1] An AAV vector comprising a capsid protein having an amino acid sequence which has at least one of serine at position 472, serine at position 587 and asparagine at position 706 in the amino acid sequence represented by SEQ ID NO:2 or 3 substituted with other amino acid, and has 1-6 amino acid residues at other residue positions deleted, substituted, inserted or added, wherein the adeno-associated virus vector is not cross-reactive with a neutralizing antibody against AAV serotype 2 present in a serum.
[2] The AAV vector according to [1] above, comprising a capsid protein having an amino acid sequence which has the serine at position 472, the serine at position 587 and the asparagine at position 706 each substituted with an amino acid selected from the group consisting of glycine, alanine, valine, leucine, threonine and isoleucine.
[3] The AAV vector according to [1] above, comprising a capsid protein having an amino acid sequence which has at least one of the serine at position 472, the serine at position 587 and the asparagine at position 706 substituted with alanine.
[4] The AAV vector according to [1] above, wherein the capsid protein comprises a protein having the amino acid sequence represented by SEQ ID NO:4.
[5] The AAV vector according to [1] above, wherein the neutralizing antibody is an antibody against an AAV of a serotype different from AAV3 or AAV8.
[6] The AAV vector according to [1] above, wherein the neutralizing antibody is an antibody against AAV2.
[7] The AAV vector according to [1] above, comprising a viral genome containing a hepatocyte-specific promoter sequence.
[8] The AAV vector according to [1] above, wherein the hepatocyte-specific promoter sequence comprises a polynucleotide having 90% or more homology with a polynucleotide sequence selected from the group consisting of an ApoE promoter, an antitrypsin promoter, a cKit promoter, a promoter for a liver-specific transcription factor (HNF-1, HNF-2, HNF-3, HNF-6, C/ERP, or DBP), a promoter for albumin or a thyroxine-binding globulin (TBG), and the polynucleotide sequence represented by SEQ ID NO:1, and serves as a liver-specific promoter.
   [8a] The AAV vector according to [1] above, comprising a therapeutic gene operably linked to the hepatocyte-specific promoter sequence.
   [8b] The AAV vector according to [1] above, wherein the therapeutic gene encodes coagulation factor VIII (FVIII), coagulation factor IX (FIX), hepatocyte growth factor (HGF) or hepatocyte growth factor receptor (c-Kit).
[9] A AAV vector comprising a capsid protein having an amino acid sequence which has at least one of serine at position 472, serine at position 587 and asparagine at position 706 in the amino acid sequence represented by SEQ ID NO:2 or 3 substituted with other amino acid, and has 1-6 amino acid residues at other residue positions deleted, substituted, inserted or added.
[10] A polynucleotide coding for any one of the following sequences:
   an amino acid sequence which has at least one of serine at position 472, serine at position 587 and asparagine at position 706 in the amino acid sequence represented by SEQ ID NO:2 or 3 substituted with other amino acid, and has 1-6 amino acid residues at other residue positions deleted, substituted, inserted or added;
   an amino acid sequence which has the serine at position 472, the serine at position 587 and the asparagine at position 706 each substituted with an amino acid selected from the group consisting of glycine, alanine, valine, leucine, threonine and isoleucine;
   an amino acid sequence which has at least one of the serine at position 472, the serine at position 587 and the asparagine at position 706 substituted with alanine; or
   the amino acid sequence represented by SEQ ID NO:4.
   [10a] The polynucleotide according to [9] above, further comprising a nucleotide sequence coding for any of the amino acid sequences represented by SEQ ID NOS:6-8.
[11] A pharmaceutical composition for use in transferring a gene into a liver of a living body, which comprises the AAV vector according to any one of [1]-[9] above.
[12] The pharmaceutical composition according to [11] above, wherein the living body is human.

### EFFECT OF THE INVENTION

The present invention provides an AAV vector which is less cross-reactive with a neutralizing antibody against AAV2 or the like and which is highly efficient in transferring a gene into hepatocytes, for example, an AAV vector derived from AAV3 or AAV8. Furthermore, the AAV vector according to the present invention can be used to perform a gene therapy to patients who have not previously been targeted by the gene therapy because they inherently possess a neutralizing antibody, to perform a gene therapy once more for patients who have not achieved a satisfactory effect by the previous gene therapy, and the like. The AAV vector of the present invention can be used to enhance gene transfer particularly into hepatocytes.

### BREIF DESCRIPTION OF DRAWINGS

Figure 1A shows the amino acid alignment of VP1 proteins of AAV3A, AAV3B and AAVGT5.
Figure 1B shows the alignment continued from Figure 1A.
Figure 1C shows the amino acid sequence alignment of Rep proteins of AAV3A, AAV3B and AAVGT5 (referred to as ARep, BRep and baRep, respectively).
Figure 1D shows the alignment continued from Figure 1C.
Figure 2A shows a GFP expression level of AAVGT5 in human liver-derived HepG2 cells.
Figure 2B shows the states of the gene-transferred, GFP-expressing cells shown in Figure 2A.
Figure 3A shows a GFP expression level of AAVGT5 in human liver-derived PXB cells.
Figure 3B shows the states of the gene-transferred, GFP-expressing cells shown in Figure 3A.
Figure 4A shows comparison of GFP intensities among AAVGT5-CMV-AcGFP, SPARK 100-CMV-AcGFP and AAVhu37-CMV-AcGFP, 9 days after the administration thereof to HepG2 cells.
Figure 4B shows appearances of the HepG2 cells 7 days after the administration of AAVGT5-CMV-AcGFP, SPARK 100-CMV-AcGFP and AAVhu37-CMV-AcGFP to the cells.
Figure 4C shows comparison of GFP intensities among AAVGT5-CMV-AcGFP, SPARK100-CMV-AcGFP and AAVhu37-CMV-AcGFP, 9 days after the administration thereof to PXB cells.
Figure 4D shows appearances of the PXB cells 10 days after the administration of AAVGT5-CMV-AcGFP, SPARK100-CMV-AcGFP and AAVhu37-CMV-AcGFP to the cells.
Figure 5A shows a schematic view of an antibody titer measurement.
Figure 5B shows results of the antibody (Ab) titers obtained in Sera 1-4. The dashed line in the figure indicates 50% of the level in Serum (-).
Figure 6A shows results obtained when each of the four sera containing a neutralizing antibody against AAV2 (Sera 1-4) was reacted with AAV3-CMV-AcGFP or AAVGT5-CMV-AcGFP, and then each of the AAV vectors was used to infect HEK293 cells to compare GFP expressions of the AAV vectors.
Figure 6B shows the states of the gene-transferred, GFP-expressing cells shown in Figure 6A.

### MODES FOR CARRYING OUT INVENTION

The present invention provides a recombinant AAV vector which improves the efficiency of transferring a gene into liver cells, a pharmaceutical composition comprising said vector, and the like.

### 1. Recombinant AAV vector according to the invention

### 1.1. Adeno-associated virus (AAV)

The AAVs comprise viruses of various known serotypes. Examples of AAVs that present tropism towards hepatocytes (hepatic parenchymal cells) include viruses of serotypes 2, 3 (3A and 3B) and 8. According to the present invention, a vector used for delivery to hepatocytes of a living body may be, for example, any of various adeno-associated virus vectors described in Patent document 1 (WO 2008/124724).

Native AAVs are nonpathogenic. Utilizing this feature, various recombinant virus vectors carrying a gene of interest have been prepared and used for gene therapies (see, for example, WO2003/018821, WO2003/053476, WO2007/001010, YAKUGAKU ZASSHI, 126 (11), 1021-1028, etc.). Wild-type AAV genome is a single-stranded DNA molecule with a total nucleotide length of about 5 kb, and is either a sense strand or an antisense strand. The AAV genome generally has inverted terminal repeat (ITR) sequences of about 145 nucleotides long at both 5' and 3' ends of the genome. The ITRs are known to have various functions, including a function as the replication origin of the AAV genome, a function as the signal for packaging the genome into virions, and the like (see, for example, aforementioned YAKUGAKU ZASSHI 126 (11) 1021-1028, etc.). The internal domain of the wild-type AAV genome located between the ITRs (hereinafter, referred to as the internal domain) contains AAV replication (rep) gene and capsid (cap) gene. The rep and cap genes encode a protein involved in viral replication (Rep) and a capsid protein forming a virus particle, i.e., a shell having a regular icosahedral structure, (e.g., at least one of VP1, VP2 and VP3), respectively. For more detail, see, for example, Human Gene Therapy, 13, pp. 345-354, 2002, Neuronal Development 45, pp. 92-103, 2001, Experimental Medicine 20, pp. 1296-1300, 2002, YAKUGAKU ZASSHI 126(11), 1021-1028, Hum Gene Ther, 16, 541-550, 2005, etc.

The rAAV vectors of the present invention are, but not limited to, vectors derived from naturally occurring adeno-associated virus serotype 1 (AAV1), serotype 2 (AAV2), serotype 3 (AAV3A/AAV3B), serotype 4 (AAV4), serotype 5 (AAV5), serotype 6 (AAV6), serotype 7 (AAV7), serotype 8 (AAV8), serotype 9 (AAV9), serotype 10 (AAV10) and serotype rh10 (AVVrh10; Hu,C. et al., Molecular Therapy vol. 22 no. 10 oct. 2014, 1792-1802). The nucleotide sequences of the genomes of these adeno-associated viruses are known and reference can be made to the nucleotide sequences under GenBank accession numbers: AF063497.1 (AAV1), AF043303 (AAV2), NC_001729 (AAV3), NC_001829.1 (AAV4), NC_006152.1 (AAV5), AF028704.1 (AAV6), NC_006260.1 (AAV7), NC_006261.1 (AAV8), AY530579 (AAV9) and AY631965.1 (AAV10), respectively.

According to the present invention, it is preferred that a capsid protein (VP1, VP2, VP3, etc.) derived from AAV2, AAV3B (AF028705.1), AAV8 or AAV9 is utilized especially for their tropism towards hepatocytes. The amino acid sequences of these capsid proteins are known and reference can be made, for example, to the sequences registered under the above-mentioned GenBank accession numbers corresponding to the respective AAVs.

### 1.2. Capsid protein according to the invention

A recombinant AAV vector used in the present invention comprises a mutated capsid protein. Such a mutant capsid protein comprises a mutant protein having an amino acid sequence which has at least one (e.g., one, preferably two, and more preferably all three) of serine at position 472, serine at position 587 and asparagine at position 706 in the amino acid sequence represented by SEQ ID NO:2 or 3 substituted with other amino acids and further has deletion, substitution, insertion or addition of a plurality of amino acid residues at positions other than the residue positions 472, 587 and 706, and which can serve as a capsid protein. Two or more of these deletion, substitution, insertion and addition may be included in combination at the same time.

Alternatively, the recombinant AAV vector used in the present invention comprises a capsid protein having an amino acid sequence which has at least one, for example one, preferably two and more preferably all three of serine at position 472, serine at position 587 and asparagine at position 706 in the amino acid sequence represented by SEQ ID NO:2 or 3 substituted with an amino acid selected from the group consisting of glycine, alanine, valine, leucine, threonine and isoleucine, preferably alanine (e.g., the amino acid sequence represented by SEQ ID NO:4) and which further has deletion, substitution, insertion or addition of a plurality of amino acid residues at positions other than the residue positions 472, 587 and 706.

The number of the above-described deleted, substituted, inserted or added amino acid residues may be, for example, 1-50, 1-40, 1-35, 1-30, 1-25, 1-20, 1-15, 1-14, 1-13, 1-12, 1-11, 1-10, 1-9 (one to several), 1-8, 1-7, 1-6, 1-5, 1-4, 1-3, 1-2 or 1. In general, the number of the above-described deleted, substituted, inserted or added amino acid residues is smaller the better.

Preferably, the mutant capsid protein of the recombinant AAV vector used in the present invention may be a protein which has an amino acid sequence having about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to any of the amino acid sequences represented by SEQ ID NOS:2-4, and which can serve as a capsid protein.

According to the present invention, a protein that can serve as a capsid protein refers to a protein which can form a virus vector possessing an infectivity to a target cell. The capsid protein used in the present invention can form a virus vector by itself or together with other capsid protein members (e.g., VP2, VP3, etc.). Inside the virus vector, a polynucleotide including a therapeutic gene of interest that is to be delivered to target cells, for example, hepatocytes, is packaged.

Preferably, a virus vector containing a mutant capsid protein has an infectivity comparable to that of a virus vector containing a wild-type capsid protein, meaning specifically that the infectivity is preferably 50%, 60%, 70%, 80%, 85%, 90%, 91%, 92%, 93%, 94%, 95%, 96%, 97%, 98%, 99% or more relative to the infectivity of a wild-type viral genome per weight. The infectivity can be measured by a method known in the art, for example, by a reporter assay.

Examples of the amino acid residues that are mutually replaceable in the protein (polypeptide) of the present invention are shown below. Amino acid residues belonging to the same group are interchangeable with each other.
Group A: Leucine, isoleucine, norleucine, valine, norvaline, alanine, 2-aminobutyric acid, methionine, o-methyl serine, t-butyl glycine, t-butyl alanine, and cyclohexylalanine;
Group B: Aspartic acid, glutamic acid, isoaspartic acid, isoglutamic acid, 2-aminoadipic acid, and 2-aminosuberic acid;
Group C: Asparagine, and glutamine;
Group D: Lysine, arginine, ornithine, 2,4-diaminobutyric acid, and 2,3-diaminopropionic acid;
Group E: Proline, 3-hydroxyproline, and 4-hydroxyproline;
Group F: Serine, threonine, and homoserine;
Group G: Phenylalanine, and tyrosine.

A protein containing an amino acid residue substitution of interest can be prepared according to a method known to those skilled in the art, for example, by a general genetic engineering technique or chemical synthesis. For such a genetic engineering procedure, see, for example, Molecular Cloning 4th Edition, J. Sambrook et al., Cold Spring Harbor Lab. Press. 2012, Current Protocols in Molecular Biology, John Wiley and Sons 1987-2018 (ISSN: 1934-3647, etc.), and the like.

The recombinant AAV virus vector used in the present invention may contain, in the packaged genome or in a genome of a helper virus, a gene encoding the Rep protein involved in replication. Such a Rep protein may have an amino acid sequence that preferably have about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more or 99.9% or more identity to the wild-type Rep protein, as long as it has the function of recognizing the ITR sequences to carry out genome replication depending on said sequences so that the recombinant AAV of the present invention is replicated, the function of packaging the wild-type AAV genome (or the recombinant AAV genome) into the virus vector, and the function of forming the recombinant AAV vector of the present invention. Alternatively, it may include deletion, substitution, insertion and/or addition of 60 or less, 50 or less, 40 or less, 30 or less, 20 or less, 15 or less, 10 or less or 5 or less amino acid residues. Herein, "comparable to wild-type" means that the specific activity relative to the wild type is 50%, 60%, 70%, 80%, 90% or more. In the present invention, a Rep protein from a known AAV3, among others, for example, a Rep protein from AAV3A or AAV3B, or a fusion protein thereof (the amino acid sequence represented by SEQ ID NO:8) or the like can be preferably used, while the present invention is not limited thereto.

In one embodiment of the present invention, the above-described capsid protein VP1 or others (VP1, VP2 and/or VP3) and Rep protein encoded in the internal domain of the wild-type AAV genome are used for incorporating polynucleotides coding for these proteins into an AAV helper plasmid, and for obtaining a recombinant AAV of the present invention. If necessary, the capsid protein (VP1, VP2 and/or VP3) and the Rep protein used in the present invention may be incorporated into one, two, three or more kinds of plasmids. In some cases, one or more kinds of these capsid proteins and Rep proteins may be contained in the AAV genome. In the present invention, all of the capsid protein (VP1, VP2 and/or VP3) and the Rep protein are preferably encoded together by one strand of polynucleotide, and provided as an AAV helper plasmid.

### 1.3. Viral genome

### (1) recombinant AAV viral genome

The polynucleotide packaged into the AAV vector of the present invention (i.e., the polynucleotide) can be prepared by substituting a polynucleotide of the internal domain located between the ITRs at the 5' and 3' of the wild-type genome (i.e., either or both of rep gene and cap gene) with a gene cassette containing a polynucleotide encoding the protein of interest (genome editing means and/or repair gene), a promoter sequence for transcription of said polynucleotide, and others. Preferably, the ITRs at the 5' and 3' are located at the 5' and 3' ends of the AAV genome, respectively. The ITRs at the 5' and 3' ends of the recombinant AAV genome of the present invention preferably include, but not limited to, the 5' ITR and the 3' ITR contained in the genome of AAV1, AAV2, AAV3, AAV6, AAV8 or AAV9. Since the ITR parts generally have sequences with easily stitched complementary sequences (flip and flop structure), the 5'-to-3' orientation of one ITR contained in the recombinant AAV genome of the present invention may be inverted. In the recombinant AAV genome of the present invention, the polynucleotide that replaces the internal domain (namely, the genome editing means and/or the repair gene) preferably has a practical length that is substantially equal to the length of the original polynucleotide. Specifically, the total length of the recombinant AAV genome of the present invention is substantially equal to the total length of the wild-type, i.e., 5 kb, for example, about 2-6 kb, preferably about 4-6 kb. The length of the therapeutic gene incorporated into the recombinant AAV genome of the present invention is preferably, but not limited to, about 0.01-3.7 kb, more preferably about 0.01-2.5 kb and still more preferably 0.01-2 kb, except the length of the transcription regulatory region including the promoter, the polyadenylation site and others (assuming, for example, said length is about 1-1.5 kb). Furthermore, as long as the total length of the recombinant AAV genome is within the above-mentioned range, two or more therapeutic genes can be incorporated by using a known technique in the art, such as interposition of a known internal ribosome entry site (IRES) sequence, T2A sequence or the like. When the recombinant AAV of the present invention expresses two or more proteins, the genes encoding these proteins may have the same or different orientation.

In general, if the polynucleotide packaged into the recombinant adeno-associated virus vector is a single strand, it may take time (several days) for the gene of interest (therapeutic gene, etc.) to be expressed. In this case, the gene of interest to be introduced is designed to take a self-complementary (sc) structure to shorten the time for expression. Specifically, see, for example, Foust K.D. et al. (Nat Biotechnol. 2009 Jan; 27 (1): 59-65), etc. The polynucleotide packaged into the AAV vector of the present invention may have either a non-sc structure or a sc structure. Preferably, the polynucleotide packaged into the AAV vector of the present invention has a non-sc structure.

The capsid protein used in the present invention may be encoded, for example, by a polynucleotide suitably modified based on the codon priority in the host cell. The polynucleotide encoding a preferred capsid protein used in the present invention comprises, for example, a polynucleotide which has deletion, substitution, insertion and/or addition of one or more (e.g., 1-50, 1-40, 1-30, 1-25, 1-20, 1-15, 1-10, 1-9 (one to several), 1-8, 1-7, 1-6, 1-5, 1-4, 1-3, 1-2, 1, etc.) nucleotides in a polynucleotide sequence coding for any of the amino acid sequences represented by SEQ ID NOS:2-4, and which codes for a protein containing any of the amino acid sequences represented by SEQ ID NOS:2-4 or a protein including deletion, substitution, insertion and/or addition of one or more amino acids mentioned above in any of the amino acid sequences represented by SEQ ID NOS:2-4, serving as a capsid protein (i.e., which can form a capsomere). Two or more of these deletion, substitution, insertion and addition can be included in combination at the same time. In general, the number of the above-described deleted, substituted, inserted or added nucleotides is smaller the better. Moreover, a preferred polynucleotide according to the present invention includes, for example, a polynucleotide coding for any of the amino acid sequences represented by SEQ ID NOS:2-4 or a polynucleotide which can hybridize to the complementary sequence thereof under stringent hybridization conditions and which codes for a protein containing any of the amino acid sequences represented by SEQ ID NOS:2-4 or a protein including deletion, substitution, insertion and/or addition of one or more of the above-mentioned amino acids in the amino acid sequences represented by SEQ ID NOS:2-4, coding for a capsid protein.

Hybridization can be carried out according to a known method or a method corresponding thereto, for example, a method described in Molecular Cloning (Molecular Cloning 4th Edition, J. Sambrook et al., Cold Spring Harbor Lab. Press. 2012). In a case where a commercially available library is employed, hybridization can be carried out, for example, according to the method described in the instruction provided by the manufacturer. Herein, "stringent conditions" may be any of lowly, moderately or highly stringent conditions. The "lowly stringent conditions" refer to, for example, the condition including 5 × SSC, 5 × Denhardt's solution, 0.5% SDS and 50% formamide at 32°C. The "moderately stringent conditions" refer to, for example, the condition including 5 × SSC, 5 × Denhardt's solution, 0.5% SDS and 50% formamide at 42°C. The "highly stringent conditions" refer to, for example, the condition including 5 × SSC, 5 × Denhardt's solution, 0.5% SDS and 50% formamide at 50°C. Under these conditions, highly homologous DNA is expected to be obtained more efficiently at a higher temperature. It is considered that the hybridization stringency may depend on multiple factors including the temperature, the probe concentration, the probe length, the ionic strength, the time, the salt concentration and the like, and thus those skilled in the art can appropriately select these factors to achieve similar stringency.

Examples of a hybridizable polynucleotide can include a polynucleotide having, for example, 70% or more, 80% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, 99.9% or more identity in comparison with a control polynucleotide sequence, as calculated by a homology search software such as FASTA and BLAST using default parameters. In general, the value of the above-described homology is higher the more preferable.

The identity or the homology of the amino acid sequence or the polynucleotide sequence can be determined using the BLAST algorithm of Karlin and Altschul (Proc. Natl. Acad. Sci. USA 872264-2268, 1990; Proc. Natl. Acad. Sci USA 90: 5873, 1993). Programs called BLASTN and BLASTX derived from the BLAST algorithm have been developed (Altschul SF, et al: J Mol Biol 215: 403, 1990). If the BLASTN is used to analyze the base sequence, the parameters are set to, for example, as follows: Expect threshold = 100, Word size = 12. Furthermore, if the BLASTX is used to analyze the amino acid sequence, the parameters are set to, for example, as follows: Expect threshold = 50, Word size = 3. If the BLAST and Gapped BLAST programs are used, the default parameters of each program are preferably used, which may appropriately be altered within a range known to those skilled in the art.

### 2. Cross-reactivity of neutralizing antibody

The adeno-associated virus vector according to the present invention is not cross-reactive with a neutralizing antibody against AAV serotype 2 (AAV2) or AAV serotype 8 (AAV8) present in a serum.

As used in the present invention, a neutralizing antibody refers to an antibody which binds to an antigen which may have an activity, including toxicity or infectivity to a living body, thereby decreasing or eliminating said activity. In the present invention, the term "neutralization" in the context of activity of an antibody means, for example, that an anti-adeno-associated virus neutralizing antibody (e.g., IgG, IgM, IgA) in a serum binds to the AAV vector, i.e., the antigen, resulting in reduction or elimination of the gene transfer ability of said AAV vector. This neutralization reaction comprises a series of complex reactions including binding between the antigen (e.g., the AAV vector) and the antibody, binding between the Fc portion of the antibody and the first complement component (C1), and the like, which results in reduction or elimination of the infectivity (or gene transfer ability) of the AAV vector to the target cell. Moreover, in the present invention, it is intended that a neutralizing antibody is accompanied with a serum component; specifically, a neutralizing antibody may contain a component, such as a complement, that is bound to the antibody and directly involved in the neutralization reaction, including inactivation or removal of the antigen.

In the present invention, cross reactivity of a neutralizing antibody refers to a reaction where the neutralizing antibody binds to a target substance (e.g., AAV3) different from the originally targeted antigen (e.g., AAV2). Further, as used in the present invention, the phrase "not cross-reactive with a neutralizing antibody" means that the neutralizing antibody does not function sufficiently in binding (preferably does not cause any detectable binding) to a target substance other than the originally intended target antigen, or means that, even if the binding is detectable, the amount of the antibody bound to the target substance is significantly reduced (in a mole ratio or mass ratio), as compared with the amount of the antibody bound to the originally intended target antigen. Such a reduced amount of the antibody bound to the target substance may be, for example, several % (about 5%), about 10%, about 15%, about 20%, about 25%, about 30%, about 35%, about 40%, about 45%, about 50% or about 60% relative to the amount of the antibody bound to the originally targeted antigen. Such a ratio can be determined by using a known method, such as a method for measuring an amount of a protein bound to an antibody. Alternatively, cross reactivity of a neutralizing antibody can also be measured and compared indirectly by using a protein encoded by the recombinant virus (such as a reporter gene).

A neutralizing antibody according to the present invention is preferably derived from a mammal, more preferably from a primate and most preferably from a human. Unless otherwise specified, the neutralizing antibody used in the present invention refers to a neutralizing antibody against AAV2. Usually, it is highly likely that a human has antibodies against AAV2, while majority of the antibodies do not have neutralizing ability. There are several reports describing that the proportion of the antibodies having neutralizing ability is 18-32% (Chirmule N. et al., Gene Ther 6: 1574-1583, 1999; Moskalenko M, et al., J Virol 74: 1761-1766, 2000). Furthermore, it is known in the art that most of the human sera containing neutralizing antibodies against AAV2 also contain neutralizing antibodies against AAV3A and AAV3B (Fu H et al., Hum Gene Ther Clin Dev 2017; 28:187-196, Ling CJ et al., Integr Med 2015;13:341-346). This is also suggested in that there is a relatively high amino acid identity (specifically, about 87-88%) between the AAV2 VP1 protein (SEQ ID NO:1) and the AAV3A or AAV3B VP1 protein (SEQ ID NO:2 or 3).

Furthermore, a neutralizing antibody used in the present invention may comprise multiple isotypes (e.g., IgG, IgM, IgA, etc.). Therefore, an anti- AAV2 neutralizing antibody according to the present invention needs to be validated in advance for its performance including an antibody concentration or an antibody titer. Procedures for such validation are known in the art. For example, a procedure described in Ito et al. (Ito et al., Ann Clin Biochem 46: 508-510, 2009) can be employed. Specifically, the publication describes that a titer of a neutralizing antibody (antibody titer) was analyzed by assessing the potency of inhibiting the introduction of an AAV2 vector containing β-galactosidase reporter gene into HEK293 cells (Ito et al. above, Method).

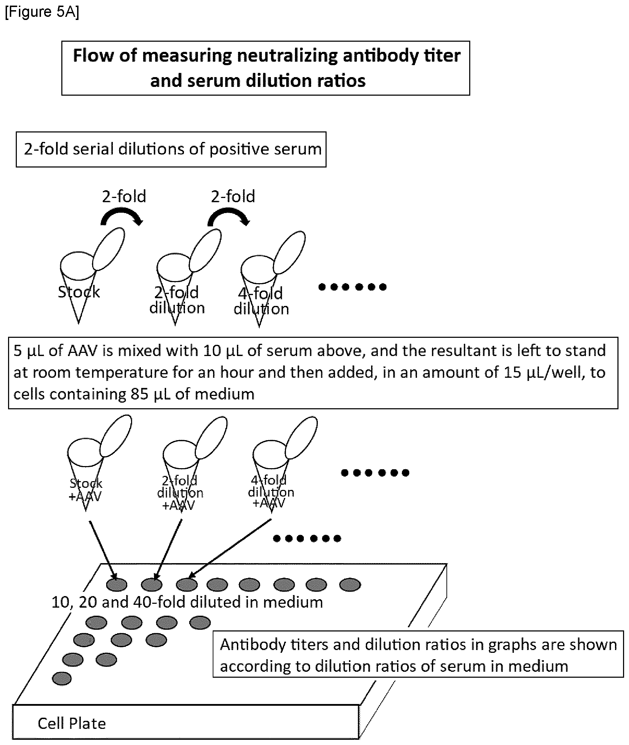

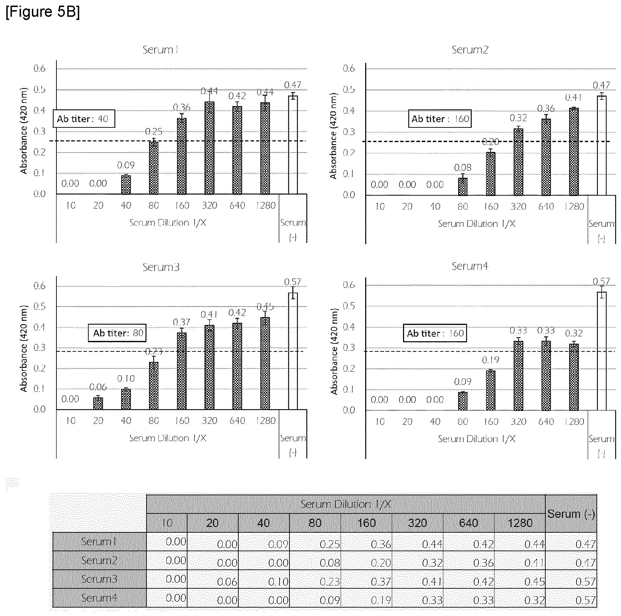

The antibody titer of the neutralizing antibody used in the present invention is 4-640, preferably 20-640, 40-320, and more preferably 40-160 (Ito et al., Ann Clin Biochem, 46: 508-510, 2009). For a specific method for measuring the antibody titer in the present invention, see the descriptions under the sections in the present specification "(e) Measurement of antibody titer of AAV2 neutralizing antibody in serum" and "(3) Measurement of antibody titer of AAV2 neutralizing antibody" in EXAMPLES, Figures 5A and 5B, and others.

In addition, as the procedures of an assay for validating a cross-reactivity of a neutralizing antibody, the method described in Li C, et al., Gene Ther 19: 288-294, 2012, Meliani A, et al., Hum Gene Ther Methos 26:45-53, 2015 or others can be also employed specifically.

The AAV vector of the present invention is less susceptible to the neutralizing antibody in the serum as compared with a wild type virus. Specifically, the virus vector of the present invention (e.g., one including a capsid protein having the amino acid sequence represented by SEQ ID NO:4) maintains at least 60% ability of transferring a gene into target cells, preferably 70% or more, more preferably 75% or more, still more preferably 80% or more, 85% or more, 90% or more, or 95% or more ability, after the treatment with a neutralizing antibody in a serum. Here, for comparison of the gene transfer ability, a known assay can be employed, such as reporter assay, *in situ* hybridization or radioisotope labeling. On the other hand, after a wild-type virus vector (e.g., one including a capsid protein having the amino acid sequence represented by SEQ ID NO:2 or 3) is treated with the neutralizing antibody in the serum in a similar manner, it maintains less than about 60%, less than about 50%, less than about 40%, less than about 35% or less than about 30% ability of transferring a gene into target cells.

In other words, the virus vector of the present invention is capable of transferring a gene into a target cell in an amount of 1.2 times or more, preferably 1.5 times or more, more preferably 1.75 times or more, and still more preferably twice or more as compared with a wild-type virus vector, after the treatment with a neutralizing antibody in a serum.

The AAV2 neutralizing antibody in the present invention may also have a neutralizing activity against AAV3 due to cross-reactivity. In addition, a serum containing the AAV2 neutralizing antibody may also be cross-reactive to other AAV serotypes (Fu H et al., Hum Gene Ther Clin Dev 2017; 28: 187-196, Ling CJ et al., Integr Med 2015; 13: 341-346, Mimuro J, et al., J Med Virol 86: 1990-1997, 2014.) Herein, the identity between the amino acid sequences of AAV2 capsid protein VP1 and AAV3A VP1 is calculated to be 87%, and the identity between the amino acid sequences of AAV2 VP1 and AAV3B VP1 is calculated to be 88% (results acquired referring to the website of Blastp (https://blast.ncbi.nlm.nih.gov/Blast.cgi)). Furthermore, studies relating to epitope mapping of an anti-AAV2 neutralizing antibody is described in Moskalenko, M. et al. (J Virol 74: 1761-1766, 2000).

The followings are known as extracellular receptors involved in infection of cells with AAV (Summerfold et al., Mol Ther 24: 2016; Pillay et al., Nature 530:108-112, 2016).

### Primary receptors

AAV2, 3, 6: Heparan sulfate proteoglycan
AAV9: Terminal N-linked galactose
AAV1, 4, 5, 6: Specific N-linked or O-linked sialic acid moiety

### Secondary receptors

AAV2: Fibroblast growth factor receptor and integrin
AAV2, 3: Hepatocyte growth factor receptor (c-Met)
AAV5: Platelet-derived growth factor (which may be modified with sialic acid)

In light of the disclosure of the present application and the findings relating to the above-described receptors, a recombinant vector which is less susceptible to inhibition by a neutralizing antibody against AAV2 or the like in a living body can be designed, screened and acquired, based on the recombinant virus vector according to the present invention.

### 3. Genes contained in virus vector of the present invention

In one embodiment, the AAV vector of the present invention preferably comprises a polynucleotide containing a liver-specific promoter sequence and a gene of interest operably linked to said promoter sequence (specifically, such a polynucleotide is packaged). The promoter sequence used in the present invention may be a promoter sequence specific to cells in the liver, for example, a promoter sequence specific to hepatic parenchymal cells, nonparenchymal cells (hepatic stellate cells, etc.) or the like. Examples of such a promoter sequence specifically include, but not limited to, an ApoE promoter, an antitrypsin promoter, a cKit promoter, a promoter for liver-specific transcription factor (HNF-1, HNF-2, HNF-3, HNF-6, C/ERP, DBP, etc.), a thyroxine-binding globulin (TBG) promoter (Ran FA, et al., Nature 520 (7546): 186-91, 2015), promoters for other liver-specific proteins (albumin, etc.), and a synthetic promoter obtained by combining the above promoters. Furthermore, these promoters can be combined with a known enhancer sequence, preferably a liver-specific enhancer sequence. Examples of such an enhancer sequence include an ApoE enhancer sequence. One or more of the promoter sequences and the enhancer sequences can be used solely or in combination. Alternatively, a synthetic promoter that utilizes the above-described liver-specific promoter and enhancer can also be used. The rAAV vector of the present invention preferably comprises a sequence of a liver-specific, more preferably, a hepatocyte (hepatic parenchymal cell)-specific ApoE promoter, antitrypsin promoter, TBG promoter or HCRhAAT synthetic promoter that is known.

The liver-specific promoter sequence used in the present invention may also be a polynucleotide which has one or more (e.g., 1-100, 1-50, 1-40, 1-30, 1-25, 1-20, 1-15, 1-10, 1-9 (one to several), 1-8, 1-7, 1-6, 1-5, 1-4, 1-3, 1-2, 1, etc.) nucleotides in the polynucleotide sequence of the above-described promoter deleted, substituted, inserted and/or added, and which can serve as a liver-specific promoter sequence. Herein, the phrase "serving as a liver-specific promoter sequence" means that when, for example, liver-derived cells and non-liver-derived cells are compared in a reporter assay, the non-liver-derived cells express the reporter at around the detection threshold or express the reporter at about 25% or less, about 20% or less, about 15% or less, about 10% or less relative to that of the liver-derived cells reporter. In addition, it is meant that a polynucleotide having the above-described deletion, substitution, insertion and/or addition has a specific activity of 50%, 60%, 70%, 80%, 90% or more relative to the original promoter sequence. The number of the above-described mutations is smaller the better.

Examples of a disease targeted by a treatment using the AAV vector of the present invention include hemophilia, acute intermittent porphyria, ornithine transcobalamin deficiency, Wilson's disease, phenylketonuria and familial hypercholesterolemia, which involve genomic disorders of hepatocytes. For example, since hemophilia A is caused by deficiency or abnormality of coagulation factor VIII (also referred to as FVIII) while hemophilia B is caused by deficiency or abnormality of coagulation factor IX (also referred to as FIX), it can be contemplated as an approach to supplement the deficiency or abnormality with the coagulation factor VIII or IX in a gene therapy.

To perform the above-described therapy, the recombinant virus vector used in the present invention can contain any of a variety of therapeutic genes. Examples of a protein encoded by such a therapeutic gene include, but not limited to, proteins such as coagulation factor VIII (FVIII), coagulation factor IX (FIX), hepatocyte growth factor (HGF) and hepatocyte growth factor receptor (c-Kit). In addition, a therapeutic gene used in the present invention may be a gene for inhibiting a function of a target (antisense nucleotides, CAS9, etc.). Examples of such a gene include, but not limited to, antisense nucleotides of a gene coding for thrombin, protein C or protein S. When used in the present invention, antisense nucleotides refer to a polynucleotide for altering (e.g., disrupting or reducing) a function of a targeted endogenous gene, or a polynucleotide for altering (e.g., reducing) the expression level of an endogenous protein. Examples of such a polynucleotide include, but not limited to, an antisense molecule, a ribozyme, interfering RNA (iRNA), microRNA (miRNA) and sgRNA. Methods for preparing and using a double-stranded RNA (dsRNA, siRNA, shRNA or miRNA) are known from a large number of literatures (see, Japanese Patent Application National Publication No. 2002-516062; US 2002/086356A; Nature Genetics, 24(2), 180-183, 2000 Feb., etc.). Furthermore, the recombinant virus vector used in the present invention may contain one or more therapeutic genes.

### 4. Pharmaceutical composition

In another embodiment of the present invention, a pharmaceutical composition containing the recombinant AAV vector (recombinant AAV virion) of the present invention is provided. The pharmaceutical composition containing the recombinant AAV vector of the present invention (hereinafter, referred to as the pharmaceutical composition of the present invention) can be used to transfer a therapeutic gene into liver cells of a subject in a highly efficient manner, thereby providing a pharmaceutical composition which can treat a disease of interest through expression of the introduced therapeutic gene. The pharmaceutical composition of the present invention comprises the recombinant AAV vector containing such a therapeutic gene. Examples of such a therapeutic gene include, but not limited to, the above-mentioned genome editing means and genome repairing means.

In one embodiment, the recombinant AAV vector of the present invention preferably contains a promoter sequence specific to liver cells, and a gene operably linked to said promoter sequence. The recombinant AAV vector of the present invention can contain a gene effective for the treatment of hemophilia so that such a gene can be transferred into cells of a liver, preferably into hepatic parenchymal cells.

In use, the pharmaceutical composition of the present invention may be administered, for example, orally, parenterally (intravenously), intramuscularly, through oral mucosa, rectally, intravaginally, transdermally, intranasally, by inhalation, or the like, and, among others, preferably parenterally and more preferably intravenously administered. The active ingredient or ingredients of the pharmaceutical composition of the present invention can be solely or added in combination, a pharmaceutically acceptable carrier or additive for a pharmaceutical preparation may be added thereto to provide the composition in a form of a pharmaceutical preparation. In this case, the pharmaceutical preparation can contain the active ingredient of the present invention in an amount of, for example, 0.1-99.9 wt%.

While the active ingredient or ingredients of the pharmaceutical composition of the present invention may be solely or added in combination, a pharmaceutically acceptable carrier or additive for a pharmaceutical preparation can be added thereto to provide the composition in a form of a pharmaceutical preparation. In this case, the active ingredient of the present invention is contained in the pharmaceutical preparation in an amount that gives, for example, a titer of 10⁵-10¹⁶ vg/mL (900 fg/mL-90 mg/mL), a titer of 10⁶-10¹⁵ vg/mL (9.0 pg/mL-9.0 mg/mL), a titer of 10⁷-10¹⁴ vg/mL (90 pg/mL-900 µg/mL), a titer of 10⁸-10¹³ vg/mL (900 pg/mL-90 µg/mL), a titer of 10⁹-10¹² vg/mL (9 ng/mL-9 µg/mL), or a titer of 10¹⁰-10¹¹ vg/mL (90 ng/mL-900 ng/mL). The pharmaceutically acceptable carrier or additive to be used may be an excipient, a disintegrant, a disintegration aid, a binder, a lubricant, a coating agent, a dye, a diluent, a dissolution agent, a dissolution aid, a tonicity-adjusting agent, a pH regulator, a stabilizer or the like.

Examples of the pharmaceutical preparation suitable for parenteral administration include an injection and a suppository. For parenteral administration, a solution obtained by dissolving the active ingredient of the present invention in either sesame oil or peanut oil or in an aqueous propylene glycol solution can be used. The aqueous solution should be appropriately buffered in need (preferably, pH 8 or higher), but firstly the liquid diluent must be isotonic. For example, physiological saline can be used as such a liquid diluent. The prepared aqueous solution is suitable for an intravenous injection, whereas the prepared oily solutions are suitable for an intraarticular injection, an intramuscular injection and a subcutaneous injection. All of these solutions can be easily produced under sterile conditions by standard pharmaceutical techniques well known to those skilled in the art. Furthermore, the active ingredient (or ingredients) of the present invention can also be applied topically to the skin. In this case, the topical administration desirably takes place in a form of cream, gel, paste or an ointment according to a standard pharmaceutical practice.

Examples of the pharmaceutical preparation suitable for oral administration include powder, a tablet, a capsule, fine granules, granules, a liquid agent or a syrup. For oral administration, any of various excipients such as microcrystalline cellulose, sodium citrate, calcium carbonate, dipotassium phosphate or glycine may be used together with starch, preferably starch of corn, potato or tapioca, any of various disintegrants such as alginic acid or a particular double salt of silicic acid, and a granulation binder such as polyvinylpyrrolidone, sucrose, gelatin or gum arabic.

The dose of the pharmaceutical composition of the present invention is not particularly limited, and a suitable dose can be selected depending on various conditions including the type of the disease, age and symptoms of the patient, the administration route, the therapeutic goal, the presence of medicine combined therewith, and the like. A daily dosage of the pharmaceutical composition of the present invention is, for example, but not limited to, 1-5000 mg, preferably 10-1000 mg per adult (e.g., body weight 60 kg). Such a dosage may be divided and given to a patient in two to four doses a day. Where vg (vector genome) is used as the dose unit, the dose can be selected from, for example, but not limited to, a range of 10⁶-10¹⁴ vg, preferably 10⁸-10¹³ vg and more preferably 10⁹-10¹² vg per kg body weight.

### 5. Administration of virus vector of the present invention

The virus vector of the present invention can be administered to a subject preferably by peripheral administration for safer and easier administration. As used herein, peripheral administration refers to administration routes commonly recognized as peripheral administrations by those skilled in the art, including intravenous administration, intra-arterial administration, intraperitoneal administration, intracardiac administration and intramuscular administration.

When administered to a subject, the virus vector of the present invention infects cells in the liver of the subject and provide a genome editing means that is delivered to the infected cells by the virus, thereby performing genome editing. The virus vector of the present invention preferably infects hepatic parenchymal cells to perform genome editing. The virus vector of the present invention preferably provides 70% or more, 80% or more, 85% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, 99.9% or more or 100% of the hepatic parenchymal cells in the liver of the subject administered that are subjected to genome editing.

### 6. Kit for preparing recombinant AAV vector of the present invention

In another embodiment of the present invention, a kit for preparing recombinant AAV of the present invention is provided. Such a kit comprises, for example, (a) a first polynucleotide for expressing a capsid protein VP1 and the like, and (b) a second polynucleotide to be packaged into the recombinant AAV vector. The first polynucleotide comprises, for example, a polynucleotide coding for amino acids represented by SEQ ID NOs. The second polynucleotide, for example, may or may not comprise a therapeutic gene of interest, and preferably comprises various restriction enzyme cleavage sites for incorporating such a therapeutic gene of interest.

The kit for preparing the recombinant AAV virion of the present invention may further comprise any component described herein (e.g., AdV helper, etc.). In addition, the kit of the present invention may further comprise instructions describing the protocols for preparing the recombinant AAV virion using the kit of the present invention.

### 7. Other terms

The terms used herein have the following meanings. For terms that are not particularly explained herein are intended to have the meanings within the scope generally understood by those skilled in the art.

As used herein, the terms "virus vector", "virus virion" and "virus particle" are used interchangeably unless otherwise indicated. In addition, the term "adeno-associated virus vector" includes recombinant adeno-associated viruses.

As used herein, the term "polynucleotide" is used interchangeably with "nucleic acids," "gene" or "nucleic acid molecule," and is intended to refer to a nucleotide polymer. As used herein, the term "nucleotide sequence" is used interchangeably with "nucleic acid sequence" or "base sequence" and is indicated by a sequence of deoxyribonucleotides (abbreviated as A, G, C, and T). For example, a "polynucleotide comprising the nucleotide sequence represented by SEQ ID NO: 1 or a fragment thereof" is intended to refer to a polynucleotide comprising a sequence represented by the respective deoxynucleotides A, G, C and/or T of SEQ ID NO: 1, or a fragment thereof.

Each of "viral genome" and "polynucleotide" according to the present invention may exist in a form of DNA (e.g., cDNA or genomic DNA) or, in some cases, in a form of RNA (e.g., mRNA). Each of the viral genome and the polynucleotide as used herein may be a double-stranded or single-stranded DNA. The single-stranded DNA or RNA may be a coding strand (also known as a sense strand) or a non-coding strand (also known as an antisense strand).

Unless otherwise specified, when a promoter, a gene of interest, a polyadenylation signal and others encoded by the recombinant AAV genome are described with respect to their locations in the gene, the strand itself is described if the recombinant AAV genome is a sense strand and its complementary strand is described if it is an antisense strand. As used herein, "r" representing "recombination" may be omitted if it is apparent from the context.

As used herein, the terms "protein" and "polypeptide" are used interchangeably and intended to refer to a polymer of amino acids. The polypeptide as used herein is represented in accordance with a conventional peptide designation, in which the N-terminus (amino terminus) is indicated on the left and the C-terminus (carboxyl terminus) on the right. Partial peptides of the polypeptide of the present invention (which, may be briefly referred to herein as "partial peptides of the present invention") includes partial peptides of the polypeptide of the present invention described above, which preferably has the same properties as the properties of said polypeptide of the present invention.

As used herein, the term "plasmid" refers to any of various known gene elements, for example, a plasmid, a phage, a transposon, a cosmid, a chromosome, etc. The plasmid can be replicated in a particular host and transport gene sequences between cells. As used herein, a plasmid contains various known nucleotides (DNA, RNA, PNA and a mixture thereof) and may be a single strand or a double strand, preferably a double strand. For example, as used herein, the term "recombinant AAV vector plasmid" is intended to include a double strand formed by a recombinant AAV vector genome and its complementary strand unless otherwise specified. The plasmid used in the present invention may be linear or circular.

As use herein, the term "packaging" refers to events including preparation of a single-stranded viral genome, assembly of a coat (capsid) protein, encapsulation of a viral genome within a capsid, and the like. When an appropriate plasmid vector (normally, a plurality of plasmids) is introduced into a cell line that allows packaging under appropriate conditions, recombinant viral particles (i.e., virus virions, virus vectors) are assembled and secreted into the culture.

Terms not particularly described herein are intended to have the meanings within the scope generally understood by those skilled in the art.

### EXAMPLES

Hereinafter, the present invention will be described more specifically by means of examples, although the scope of the present invention should not be limited to the following examples.

### A. Materials and methods

### (a) AAV vectors

Six types of AAV vectors (AAV2, AAV3B, AAVGT5, AAV8, SPARK100, AAVhu37) were used.

AAVGT5 comprises three amino acid substitutions in coat proteins VP1 of AAV3A and 3B; specifically, serine (S) at position 472 was substituted with alanine (A), serine (S) at position 587 with alanine (A), and asparagine (N) at position 706 with alanine (A).

Into each of the AAV vectors, an expression cassette composed of a cytomegalovirus promoter (CMV) promoter, cDNA of green fluorescence protein (AcGFP) and SV40 poly(A) was inserted between the inverted terminal repeats (ITR) of AAV3A. For the AAV2 vector, an expression cassette into which LacZ sequence encoding β-galactosidase has been inserted instead of cDNA of green fluorescence protein (AcGFP) was also used to prepare AAV2-CMV-AcGFP and AAV2-CMV-LacZ vectors, respectively.

AAV2: GenBank Accession #NC_001401.2 (whole genome sequence)

(The amino acid sequence of AAV2 capsid protein VP1 is represented by SEQ ID NO:1)

AAV3A: GenBank Accession #U48704 (genome sequence)

(The amino acid sequence of AAV3A capsid protein VP1 is represented by SEQ ID NO:2)

AAV3B: GenBank Accession #AF028705.1 (genome sequence)

(The amino acid sequence of AAV3B capsid protein VP1 is represented by SEQ ID NO:3)

AAVGT5: (prepared according to the present application)

(The amino acid sequence of capsid protein VP1 is represented by SEQ ID NO:4)

AAV8: GenBank Accession #NC_006261 (genome sequence)

(The amino acid sequence of AAV8 capsid protein VP1 is represented by SEQ ID NO:5)

SPARK 100:
(The amino acid sequence of SPARK100 capsid protein VP1 is represented by SEQ ID NO:9)

AAVhu37: GenBank Accession #AY530600.1 (genome sequence)

(Amino acid sequence of AAVhu37 capsid protein VP1 is represented by SEQ ID NO:10)

### (b) Cell culturing

1) HEK 293 cells
   HEK293 cells were seeded at 5 × 10⁴ cells/well and cultured using a 10% fetal calf serum (FCS)-DMEM/F12 medium (Thermo Fisher Scientific) in 5% CO₂ at 37°C.
2) HepG2 cells
   HepG2 cells were seeded at 5×10⁴ cells/well and cultured using a 10% FCS-DMEM Low glucose medium (Thermo Fisher Scientific) in 5% CO₂ at 37°C.
3) PXB cells (PhoenixBio)
   PXB cells were collected from a PXB mouse liver consisting of over 90% human hepatocytes. The cells were seeded at 7 × 10⁴ cells/well and cultured using a specialized dHCGM medium (PhoenixBio) in 5% CO₂ at 37°C.

The activities of the neutralizing antibodies against AAV2 were measured in accordance with the method described in Ito T et al., Ann Clin Biochem. 46 (Pt 6): 508-510, 2009, and sera from four persons which completely inhibited LacZ expression at the concentration of the stock solution (resulting in 10-fold dilution in the culture medium) were used.

### (c) Vector infection

Each of the AAV vectors expressing GFP (AAV2-CMV-AcGFP, AAV8-CMV-AcGFP, AAV3-CMV-AcGFP and AAVGT5-CMV-AcGFP) and the AAV vector expressing β-Galactosidase (AAV2-CMV-LacZ) described in (a) was added at a concentration of 1-5 × 10⁸ vg/well to each of the cultured cells and the cells were then cultured for 2-7 days.

In addition, each of the AAV vectors expressing GFP described in (a) (AAVGT5-CMV-AcGFP, SPARK100-CMV-AcGFP and AAVhu37-CMV-AcGFP) was added to each of the cultured cells at concentrations of 5 × 10⁸, 1 × 10⁹ and 2 × 10⁹ vg/well and the cells were then cultured for 2-10 days.

### (d) Evaluation of GFP or β-Galactosidase expression

The fluorescence intensities of GFP expressed by the AAV vectors or the absorbance from β-Galactosidase assay were measured using a plate reader (Biotech Japan Corporation) to make comparisons thereof. In addition, images of typical view fields of the GFP-expressing cells were taken using a fluorescence microscope (Olympus IX83).

### (e) Measurement of antibody titers of AAV2 neutralizing antibodies in sera

The antibody titers of the neutralizing antibodies against AAV2 were measured in advance according to the method described in Ito T et al., Ann Clin Biochem. 46 (Pt 6): 508-510, 2009, and the sera from four persons which completely inhibited LacZ expression by the AAV2 vector at the concentration of the serum stock solution (resulting in 10-fold dilution in the culture medium) were used (Sera 1-4).

HEK293 cells were seeded at 5 × 10⁴ cells/well in a 96-well plate (Thermo Fisher Scientific) and cultured at 37°C in a 5% CO2 incubator for one day. Two-fold serial dilutions of the test sera (Sera 1-4) were prepared sequentially from the stock solution to 128-fold dilution. Fetal calf serum (FCS) was used for the dilution.

AAV2-CMV-LacZ was diluted with 50 mM Hepes, 150 mM NaCl (HN) buffer to 1 × 10⁸ vg/ µL, and mixed with the above-described diluted serum at 1:2 (AAV: serum volume/volume). As a control (Sample (-)), a mixture of the FCS and AAV2-CMV-LacZ was used without the test sera. Specifically, 5 µL of AAV (2 × 10⁷ vg/ µL) and 10 µL of a diluted serum per well were allowed to react at room temperature for an hour, and 15 µL/well of the reacted mixture was added to each of the cell wells containing 85 µL of a medium. The serum dilution ratios in the media at this point were 10 to 1280-fold (Figures 5A and 5B).

After culturing at 37°C in a 5% CO₂ incubator for 2 days, β-Gal assay Kit (Thermo Fisher Scientific) was used to measure the absorbance in a plate reader to evaluate the expression of β-Galactosidase.

The maximum dilution ratio of the ratios showing less than 50% of the Sample (-) well absorbance was set as the antibody titer, which was represented in a ratio of the serum dilution in the medium.

### (f) Verification of cross-reactivity to neutralizing antibody

In order to compare the cross-reactivity of AAV3 or AAVGT5 to the AAV2 neutralizing antibody, the AAV3 or AAVGT5 vector was allowed to react with a serum containing the neutralizing antibody in advance, and then the gene transferring ability was compared between these vectors based on their GFP expression levels in HEK293 cells. HEK293 cells were seeded at 5 × 10⁴ cells/well in a 96-well optical bottom plate and used on the following day as the above-described cells.

As the neutralizing antibody-positive sera, the four human sera that completely inhibited expression from AAV2 according to the results from the measurement in (e) above were used. AAV3-CMV-AcGFP and AAVGT5-CMV-AcGFP were mixed with these sera at 1:2 (volume/volume), allowed to react therewith at room temperature for an hour, and then the reacted sample was administered to cells. Specifically, 5 µL of AAV (2 × 10⁷ vg/µL) and 10 µL of the diluted serum per well were mixed and allowed to react, and the reacted sample was administered to a cell well containing 85 µL of a medium. FCS alone was mixed and allowed to react with AAV to be used as a control (No Serum). After culturing at 37°C in a 5% CO₂ incubator for three days, fluorescence intensities of GFP were measured in a plate reader (Biotech Japan Corporation) and compared based on the levels relative to that of the control.

### B. Results

### (1) Preparation of mutant AAVGT5

Synthesized DNA containing the fused Rep sequence from the Rep sequences of the adeno-associated viruses AAV3A and AAV3B, and the VP sequence of AAV3B was prepared. In the preparation, the sequence was genetically engineered to have S472A, S587A and N706A mutations in the AAV3B VP1, thereby yielding mutant adeno-associated virus AAVGT5.

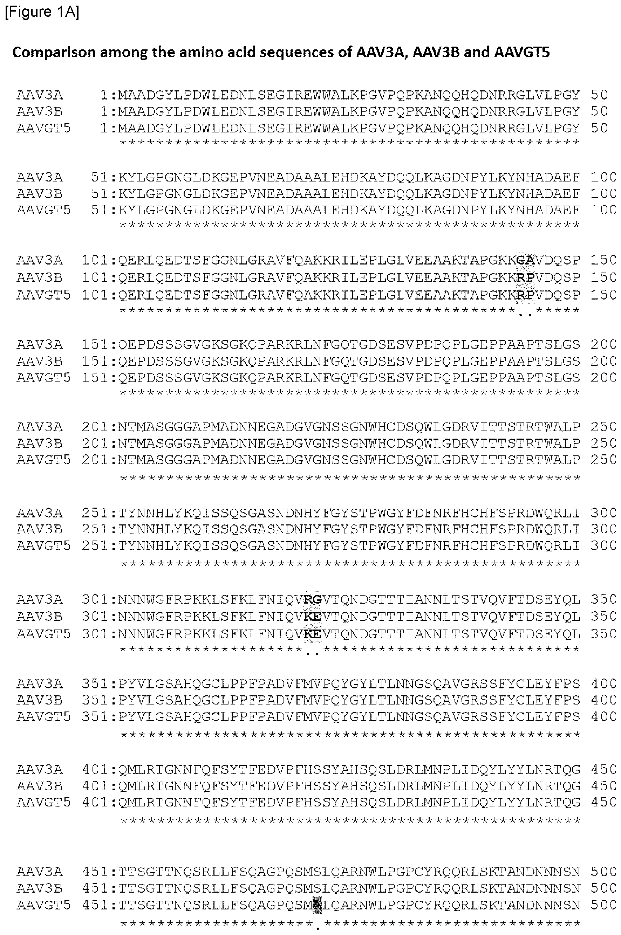

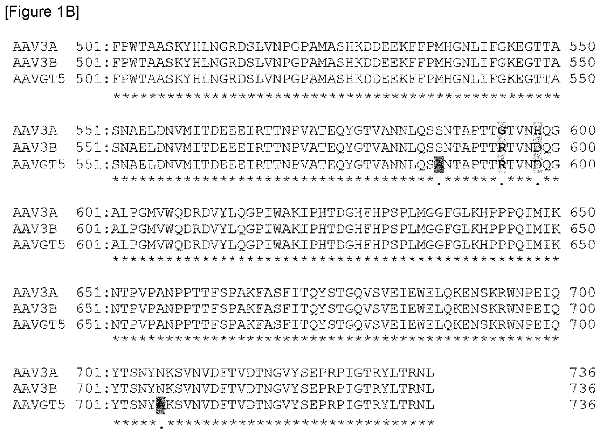

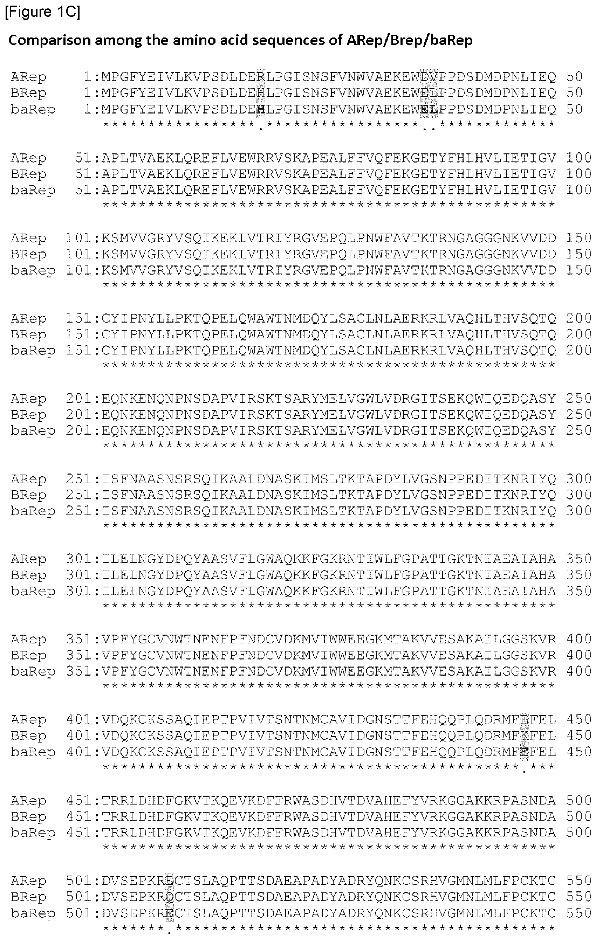

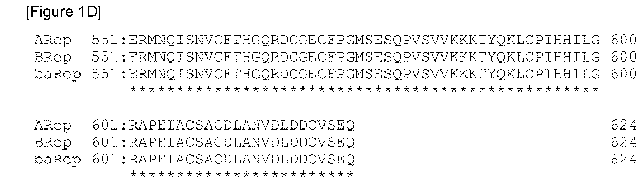

Alignments of the amino acid sequences of the VP1 proteins (SEQ ID NOs:2-4) and alignments of the amino acid sequences of the Rep proteins (SEQ ID NOs:6-8) of AAV3A, AAV3B and AAVGT5 are shown in Figures 1A-1D.

### (2) Confirmation of infectivity of AAVGT5 to human liver-derived cells HepG2 and PXB

Infectivity of AAVGT5 prepared above to human liver-derived cells was confirmed.

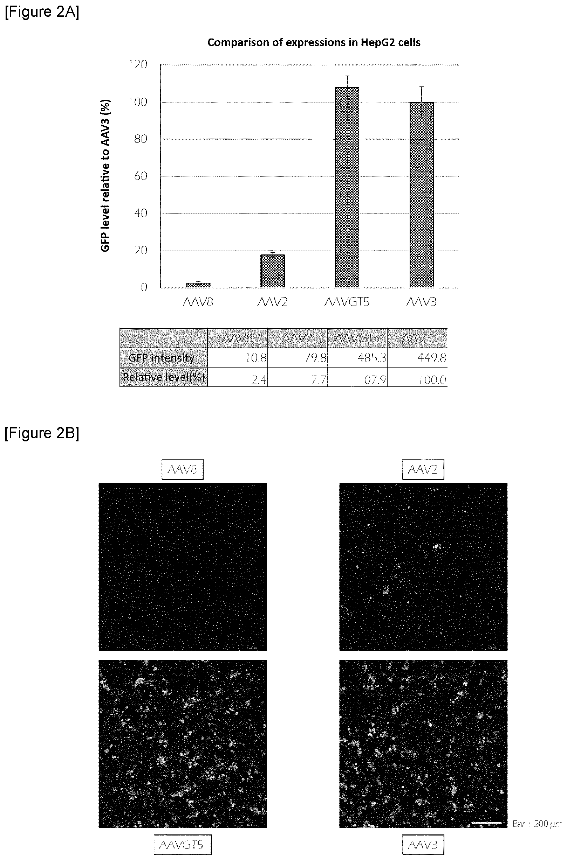

HepG2 cells, a human liver-derived cell line, were seeded at 5 × 10⁴ cells/well in a 96-well optical bottom plate, and AAV8-CMV-AcGFP, AAV2-CMV-AcGFP, AAV3-CMV-AcGFP and AAVGT5-CMV-AcGFP were each administered to the cells at a dose of 5 × 10⁸ vg/well on the following day. After culturing at 37°C in a 5% CO₂ incubator for seven days, fluorescence intensities of GFP were measured and compared in a plate reader (Figure 2A). The appearances of the cells upon the measurement are shown in Figure 2B.

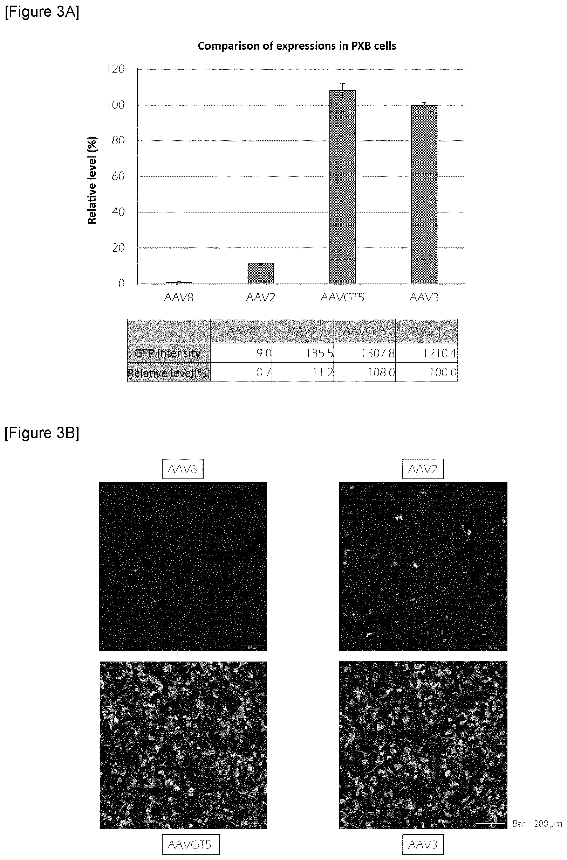

The results obtained by similarly carrying out the gene transfer into the PXB cells collected from a PXB mouse liver and composed of over 90% human hepatocytes are shown in Figure 3. Six days after 7 × 10⁴ cells/well of the PXB cells (PhoenixBio) were seeded in a 96-well plate, AAV8-CMV-AcGFP, AAV2-CMV-AcGFP, AAV3-CMV-AcGFP and AAVGT5-CMV-AcGFP were each administered thereto at a dose of 5 × 10⁸ vg/well. After culturing at 37°C in a 5% CO₂ incubator for 7 days, fluorescence intensities of GFP were measured and compared in a plate reader (Figure 3A). The appearances of the cells upon the measurement are shown in Figure 3B.

In both HepG2 cells and PXB cells, the GFP expression levels of AAVGT5-CMV-AcGFP were about 1.1 times as high as those of AAV3. It is considered that AAVGT5 can transfer a gene into these human liver-derived cells at a level comparable to or more than AAV3. On the other hand, AAV8 and AAV2 remarked lower efficiencies of gene transfer into human hepatocytes than AAV3 or AAVGT5 (Figures 2 and 3).

**Table 1: Infectivities of AAV vectors to HepG2 cells**

| | AAV8 | AAV2 | AAVGT5 | AAV3 |
|---|---|---|---|---|
| GFP intensity | 10.8 | 79.8 | 485.3 | 449.8 |
| Relative level (%) | 2.4 | 17.7 | 107.9 | 100.0 |

**Table 2: Infectivities of AAV vectors to PXB cells**

| | AAV8 | AAV2 | AAVGT5 | AAV3 |
|---|---|---|---|---|
| GFP intensity | 9.0 | 135.5 | 1307.8 | 1210.4 |
| Relative level (%) | 0.7 | 11.2 | 108.0 | 100.0 |

### (3) Confirmation of infectivity of AAVGT5 to human liver-derived cells HepG2 and PXB

Infectivity of the above-prepared AAVGT5 to human liver-derived cells was confirmed.

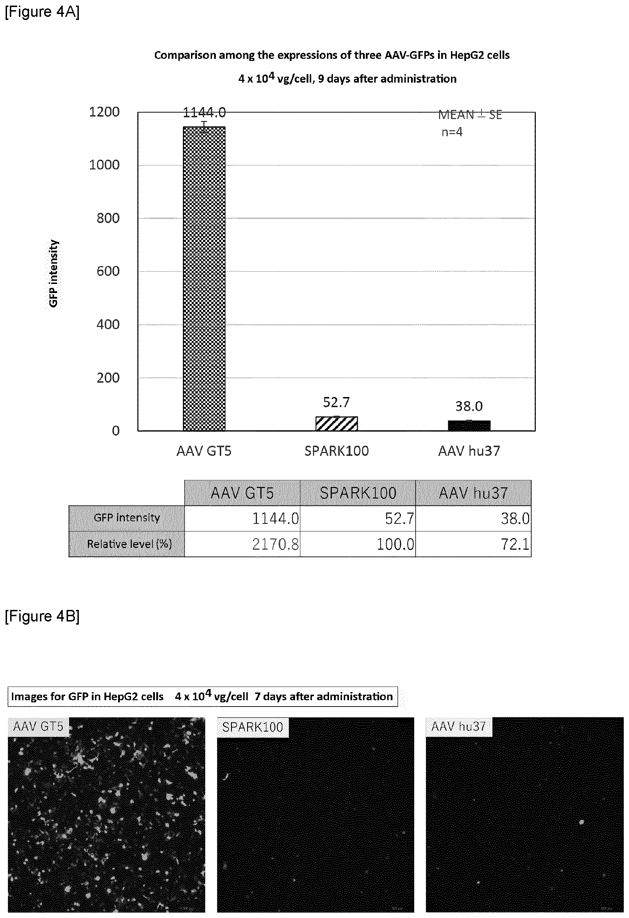

HepG2 cells, a human liver-derived cell line, were seeded at 5 × 10⁴ cells/well in a 96-well optical bottom plate, and AAVGT5-CMV-AcGFP, SPARK100-CMV-AcGFP and AAVhu37-CMV-AcGFP were each administered to the cells at a dose of 2 × 10⁹ vg/well (4 × 10⁴ vg/cell) on the following day. After culturing at 37°C in a 5% CO₂ incubator for 9 days, fluorescence intensities of GFP were measured and compared in a plate reader (Figure 4A). The appearances of the cells 7 days after the administration are shown in Figure 4B.

**Table 3: GFP expressions of AAV vectors in HepG2 cells**

| | AAVGT5 | SPARK 100 | AAVhu37 |
|---|---|---|---|
| GFP intensity | 1144.0 | 52.7 | 38.0 |
| Relative level (%) | 2170.8 | 100.0 | 72.1 |

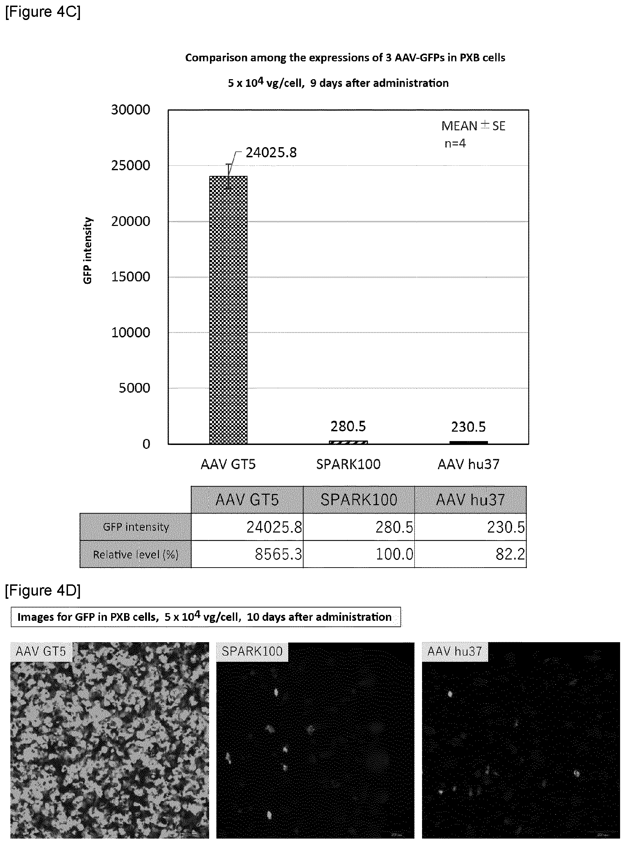

The results obtained by similarly carrying out the gene transfer into the PXB cells collected from a PXB mouse liver and composed of over 90% human hepatocytes are shown in Figure 4C. Six days after 7 × 10⁴ cells/well of the PXB cells (PhoenixBio) were seeded in a 96-well plate, AAVGT5-CMV-AcGFP, SPARK100-CMV-AcGFP and AAVhu37-CMV-AcGFP were each administered thereto at a dose of 3.5 × 10⁹ vg/well (5 × 10⁴ vg/cell). After culturing at 37°C in a 5% CO₂ incubator for 9 days, fluorescence intensities of GFP were measured and compared in a plate reader (Figure 4C). The appearances of the cells after 10 days of culture are shown in Figure 4D.

**Table 4: GFP expressions of AAV vectors in PXB cells**

| | AAVGT5 | SPARK 100 | AAVhu37 |
|---|---|---|---|
| GFP intensity | 24025.8 | 280.5 | 230.5 |
| Relative level (%) | 8565.3 | 100.0 | 82.2 |

The GFP expression level of AAVGT5-CMV-AcGFP was 22 times higher than that of SPARK100-CMV-AcGFP in the HepG2 cells, and 86 times higher in the PXB cells. It is considered that AAVGT5 can transfer a gene into these human liver-derived cells with a high efficiency.

### (4) Measurement of antibody titers of AAV2 neutralizing antibody

According to the procedure of (e) above (Figure 5A), antibody titers of the AAV2 antibodies were measured in the test sera (Sera 1-4).

Based on the results, Sera 1-4 were prepared to give antibody titers of 40, 160, 80 and 160, respectively (Figure 5B, Table 5 below). These Sera 1-4 were subjected to experiments relating to cross-reactivity.

**Table 5**

| | Serum dilution 1/x | | | | | | | | Serum (-) |
|---|---|---|---|---|---|---|---|---|---|
| | 10 | 20 | 40 | 80 | 160 | 320 | 640 | 1280 | |
| Serum 1 | 0.00 | 0.00 | 0.09 | 0.25 | 0.36 | 0.44 | 0.42 | 0.44 | 0.47 |
| Serum 2 | 0.00 | 0.00 | 0.00 | 0.08 | 0.20 | 0.32 | 0.36 | 0.41 | 0.47 |
| Serum 3 | 0.00 | 0.06 | 0.10 | 0.23 | 0.37 | 0.41 | 0.42 | 0.45 | 0.57 |
| Serum 4 | 0.00 | 0.00 | 0.00 | 0.09 | 0.19 | 0.33 | 0.33 | 0.32 | 0.57 |

### (5) Comparison of expressions of AAVs reacted with AAV2 neutralizing antibody-positive serums

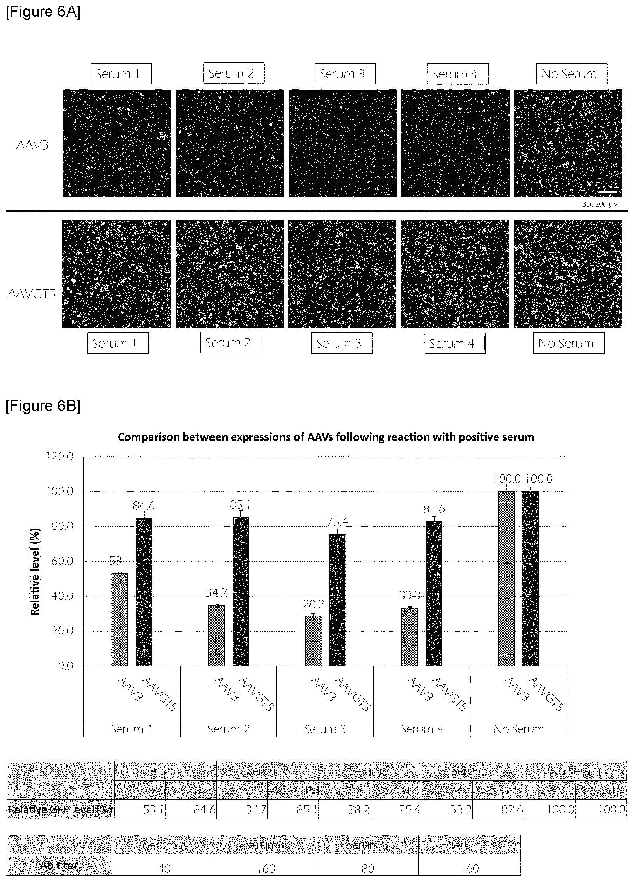

Following the reactions of AAV3-CMV-AcGFP or AAVGT5-CMV-AcGFP with the four sera containing the neutralizing antibody which completely inhibited AAV2 expression (Sera 1-4), each of the AAV vectors was used to infect HEK293 cells to compare the GFP expressions (Figure 6A).

HEK293 cells were seeded at 5 × 10⁴ cells/well in a 96-well optical bottom plate and the cells were used for the measurement of the GFP activity on the following day. Comparison was made based on the level relative to that of the control (No Serum).

The GFP expression levels of AAV3 decreased to 53-28% of that of the control by the reaction with the sera containing the high-titer neutralizing antibody (Sera 1-4), while the expression levels of AAVGT5 were maintained as high as 85-75% (Figure 6A). The apperances of the cells upon the measurement are shown in Figure 6B.

Accordingly, mutating the AAV3 vector into the AAVGT5 vector enhanced resistance to the neutralizing antibody in all four sera (specifically, cross-reactivity was reduced). As the result, the amount of a gene transferred into cells was improved.

**Table 6**

| | Serum 1 | | Serum 2 | | Serum 3 | | Serum 4 | | No serum | |
|---|---|---|---|---|---|---|---|---|---|---|
| | AAV3 | AAVGT 5 | AAV3 | AAVGT 5 | AAV3 | AAVGT 5 | AAV3 | AAVGT 5 | AAV3 | AAVG T5 |
| Relative level (%) | 53.1 | 84.6 | 34.7 | 85.1 | 28.2 | 75.4 | 33.3 | 82.6 | 100.0 | 100.0 |

### INDUSTRIAL APPLICABILITY

The recombinant adeno-associated virus vector of the present invention can reduce the attack from a neutralizing antibody of a living body so that, for example, a gene can be transferred into a target cell more efficiently. Hence, the recombinant adeno-associated virus vector of the present invention would allow more efficient gene therapy, if the vector has, for example, a liver-specific promoter and a gene for treating a hereditary disease associated with a genomic disorder of hepatocytes, such as hemophilia, thrombosis, thrombocytopenia, hereditary hemorrhagic telangiectasia, hereditary liver metabolism disorders or hepatocellular carcinoma (e.g., a gene therapy with factor VIII or IX for hemophilia or cholesterol metabolism enzyme for hyperlipidemia),.

### SEQUENCE LISTING FREE TEXT

SEQ ID NO:1: Amino acid sequence of AAV2 capsid protein
SEQ ID NO:2: Amino acid sequence of AAV3A capsid protein
SEQ ID NO:3: Amino acid sequence of AAV3B capsid protein
SEQ ID NO:4: Amino acid sequence of AAVGT5 mutant capsid protein (S472A, S587A, N706A)
SEQ ID NO:5: Amino acid sequence of AAV8 capsid protein
SEQ ID NO:6: Amino acid sequence of AAV3A Rep protein (ARep)
SEQ ID NO:7: Amino acid sequence of AAV3B Rep protein (BRep)
SEQ ID NO:8: Amino acid sequence of AAVGT5 Rep protein (baRep)
SEQ ID NO:9: Amino acid sequence of SPARK 100 capsid protein
SEQ ID NO: 10: Amino acid sequence of AAVhu37 capsid protein

## Claims

1. An adeno-associated virus vector (AAV) comprising a capsid protein having an amino acid sequence which has at least one of serine at position 472, serine at position 587 and asparagine at position 706 in the amino acid sequence represented by SEQ ID NO:2 or 3 substituted with other amino acid, and has 1-6 amino acid residues at other residue positions deleted, substituted, inserted or added, wherein the AAV vector is not cross-reactive with a neutralizing antibody against AAV serotype 2 present in a serum.

2. The adeno-associated virus vector according claim 1, comprising a capsid protein having an amino acid sequence which has the serine at position 472, the serine at position 587 and the asparagine at position 706 each substituted with an amino acid selected from the group consisting of glycine, alanine, valine, leucine, threonine and isoleucine.

3. The adeno-associated virus vector according to claim 1, comprising a capsid protein having an amino acid sequence which has at least one of the serine at position 472, the serine at position 587 and the asparagine at position 706 substituted with alanine.

4. The adeno-associated virus vector according to claim 1, wherein the capsid protein comprises a protein having the amino acid sequence represented by SEQ ID NO:4.

5. The adeno-associated virus vector according to claim 1, wherein the neutralizing antibody is an antibody against an adeno-associated virus of a serotype different from AAV3 or AAV8.

6. The adeno-associated virus vector according to claim 1, wherein the neutralizing antibody is an antibody against AAV2.

7. The adeno-associated virus vector according to claim 1, comprising a viral genome having a hepatocyte-specific promoter sequence.

8. The adeno-associated virus vector according to claim 1, wherein the hepatocyte-specific promoter sequence comprises a polynucleotide having 90% or more homology with a polynucleotide sequence selected from the group consisting of an ApoE promoter, an antitrypsin promoter, a cKit promoter, a promoter for a liver-specific transcription factor (HNF-1, HNF-2, HNF-3, HNF-6, C/ERP, or DBP), a promoter for albumin or a thyroxine-binding globulin (TBG), and the polynucleotide sequence represented by SEQ ID NO:1, and serves as a liver-specific promoter.

9. An adeno-associated virus vector comprising a capsid protein having an amino acid sequence which has at least one of serine at position 472, serine at position 587 and asparagine at position 706 in the amino acid sequence represented by SEQ ID NO:2 or 3 substituted with other amino acid and has 1-6 amino acid residues at other residue positions deleted, substituted, inserted or added.

10. A polynucleotide coding for any one of the following sequences:
an amino acid sequence which has at least one of serine at position 472, serine at position 587 and asparagine at position 706 in the amino acid sequence represented by SEQ ID NO:2 or 3 substituted with other amino acid and which has 1-6 amino acid residues at other residue positions deleted, substituted, inserted or added;
an amino acid sequence which has the serine at position 472, the serine at position 587 and the asparagine at position 706 each substituted with an amino acid selected from the group consisting of glycine, alanine, valine, leucine, threonine and isoleucine;
an amino acid sequence which has at least one of the serine at position 472, the serine at position 587 and the asparagine at position 706 substituted with alanine; or
the amino acid sequence represented by SEQ ID NO:4.

11. A pharmaceutical composition for use in transferring a gene into a liver of a living body, the composition comprising the adeno-associated virus vector according to any one of claims 1-9.

12. The pharmaceutical composition according to claim 11, wherein the living body is human.


SEQUENCE LISTING

<110>  Gene Therapy Research Institution Co.,Ltd.

<120>  AAV Virus Virion For Gene Transfer to Human Liver

<130>  G1914WO

<160>  10

<170>  PatentIn version 3.5

<210>  1
<211>  735
<212>  PRT
<213>  adeno-associated virus 2

<400>  1

<210>  2
<211>  736
<212>  PRT
<213>  adeno-associated virus 3A

<400>  2

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Val Pro Gln Pro
            20                  25                  30

Lys Ala Asn Gln Gln His Gln Asp Asn Arg Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Ile Leu Glu Pro
        115                 120                 125

Leu Gly Leu Val Glu Glu Ala Ala Lys Thr Ala Pro Gly Lys Lys Gly
    130                 135                 140

Ala Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
        180                 185                 190

Ala Ala Pro Thr Ser Leu Gly Ser Asn Thr Met Ala Ser Gly Gly Gly
    195                 200                 205

Ala Pro Met Ala Asp Asn Asn Glu Gly Ala Asp Gly Val Gly Asn Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
        260                 265                 270

Phe Gly Tyr Ser Thr Pro Trp Gly Tyr Phe Asp Phe Asn Arg Phe His
    275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Arg Gly Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
        340                 345                 350

Val Leu Gly Ser Ala His Gln Gly Cys Leu Pro Pro Phe Pro Ala Asp
    355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg

420                 425                 430

Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ser Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ser Asn Thr Ala Pro Thr
            580                 585                 590

Thr Gly Thr Val Asn His Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Met Ile Lys Asn Thr Pro Val Pro Ala
                645                 650                 655

Asn Pro Pro Thr Thr Phe Ser Pro Ala Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Asn Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Asn Leu
                725                 730                 735

<210>  3
<211>  736
<212>  PRT
<213>  adeno-associated virus 3B

<400>  3

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Val Pro Gln Pro
            20                  25                  30

Lys Ala Asn Gln Gln His Gln Asp Asn Arg Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Ile Leu Glu Pro
        115                 120                 125

Leu Gly Leu Val Glu Glu Ala Ala Lys Thr Ala Pro Gly Lys Lys Arg
    130                 135                 140

Pro Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Ala Pro Thr Ser Leu Gly Ser Asn Thr Met Ala Ser Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ser Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ser Asn Thr Ala Pro Thr
            580                 585                 590

Thr Arg Thr Val Asn Asp Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Met Ile Lys Asn Thr Pro Val Pro Ala
                645                 650                 655

Asn Pro Pro Thr Thr Phe Ser Pro Ala Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Asn Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Asn Leu
                725                 730                 735

<210>  4
<211>  736
<212>  PRT
<213>  Artificial Sequence

<220>
<223>  AAVGT5 from AAV3B

<400>  4

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Val Pro Gln Pro
            20                  25                  30

Lys Ala Asn Gln Gln His Gln Asp Asn Arg Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Ile Leu Glu Pro
        115                 120                 125

Leu Gly Leu Val Glu Glu Ala Ala Lys Thr Ala Pro Gly Lys Lys Arg
    130                 135                 140

Pro Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Ala Pro Thr Ser Leu Gly Ser Asn Thr Met Ala Ser Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ala Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ala Asn Thr Ala Pro Thr
            580                 585                 590

Thr Arg Thr Val Asn Asp Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Met Ile Lys Asn Thr Pro Val Pro Ala

                645                 650                 655

Asn Pro Pro Thr Thr Phe Ser Pro Ala Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Ala Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Asn Leu
                725                 730                 735

<210> 5
<211> 738
<212> PRT
<213> adeno-associated virus 8

<400> 5

Met Ala Ala Asp Gly Tyr Leu Pro Asp Trp Leu Glu Asp Asn Leu Ser
1               5                   10                  15

Glu Gly Ile Arg Glu Trp Trp Ala Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Gln Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg

130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Arg Lys Arg Leu Asn Phe Gly Gln
                165                 170                 175

Thr Gly Asp Ser Glu Ser Val Pro Asp Pro Gln Pro Leu Gly Glu Pro
            180                 185                 190

Pro Ala Ala Pro Ser Gly Val Gly Pro Asn Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ala Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Ser Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Gln Phe Thr Tyr
                405                 410                 415

Thr Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Thr Thr Gly Gly Thr Ala Asn Thr Gln Thr Leu Gly
    450                 455                 460

Phe Ser Gln Gly Gly Pro Asn Thr Met Ala Asn Gln Ala Lys Asn Trp
465                 470                 475                 480

Leu Pro Gly Pro Cys Tyr Arg Gln Gln Arg Val Ser Thr Thr Thr Gly
                485                 490                 495

Gln Asn Asn Asn Ser Asn Phe Ala Trp Thr Ala Gly Thr Lys Tyr His
            500                 505                 510

Leu Asn Gly Arg Asn Ser Leu Ala Asn Pro Gly Ile Ala Met Ala Thr
        515                 520                 525

His Lys Asp Asp Glu Glu Arg Phe Phe Pro Ser Asn Gly Ile Leu Ile
    530                 535                 540

Phe Gly Lys Gln Asn Ala Ala Arg Asp Asn Ala Asp Tyr Ser Asp Val
545                 550                 555                 560

Met Leu Thr Ser Glu Glu Glu Ile Lys Thr Thr Asn Pro Val Ala Thr
                565                 570                 575

Glu Glu Tyr Gly Ile Val Ala Asp Asn Leu Gln Gln Gln Asn Thr Ala
            580                 585                 590

Pro Gln Ile Gly Thr Val Asn Ser Gln Gly Ala Leu Pro Gly Met Val
        595                 600                 605

Trp Gln Asn Arg Asp Val Tyr Leu Gln Gly Pro Ile Trp Ala Lys Ile
    610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Asn Gln Ser Lys Leu Asn Ser Phe
            660                 665                 670

Ile Thr Gln Tyr Ser Thr Gly Gln Val Ser Val Glu Ile Glu Trp Glu
        675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Ser Val Asp Phe Ala Val Asn Thr Glu
705                 710                 715                 720

Gly Val Tyr Ser Glu Pro Arg Pro Ile Gly Thr Arg Tyr Leu Thr Arg
                725                 730                 735

Asn Leu

<210>  6
<211>  624
<212>  PRT
<213>  adeno-associated virus 3A

<400>  6

Met Pro Gly Phe Tyr Glu Ile Val Leu Lys Val Pro Ser Asp Leu Asp
1               5                   10                  15

Glu Arg Leu Pro Gly Ile Ser Asn Ser Phe Val Asn Trp Val Ala Glu
            20                  25                  30

Lys Glu Trp Asp Val Pro Pro Asp Ser Asp Met Asp Pro Asn Leu Ile
        35                  40                  45

Glu Gln Ala Pro Leu Thr Val Ala Glu Lys Leu Gln Arg Glu Phe Leu
    50                  55                  60

Val Glu Trp Arg Arg Val Ser Lys Ala Pro Glu Ala Leu Phe Phe Val
65                  70                  75                  80

Gln Phe Glu Lys Gly Glu Thr Tyr Phe His Leu His Val Leu Ile Glu
                85                  90                  95

Thr Ile Gly Val Lys Ser Met Val Val Gly Arg Tyr Val Ser Gln Ile
            100                 105                 110

Lys Glu Lys Leu Val Thr Arg Ile Tyr Arg Gly Val Glu Pro Gln Leu
        115                 120                 125

Pro Asn Trp Phe Ala Val Thr Lys Thr Arg Asn Gly Ala Gly Gly Gly
    130                 135                 140

Asn Lys Val Val Asp Asp Cys Tyr Ile Pro Asn Tyr Leu Leu Pro Lys
145                 150                 155                 160

Thr Gln Pro Glu Leu Gln Trp Ala Trp Thr Asn Met Asp Gln Tyr Leu
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Gln Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Ser Lys
            260                 265                 270

Ile Met Ser Leu Thr Lys Thr Ala Pro Asp Tyr Leu Val Gly Ser Asn
        275                 280                 285

Pro Pro Glu Asp Ile Thr Lys Asn Arg Ile Tyr Gln Ile Leu Glu Leu
    290                 295                 300

Asn Gly Tyr Asp Pro Gln Tyr Ala Ala Ser Val Phe Leu Gly Trp Ala
305                 310                 315                 320

Gln Lys Lys Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Glu Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Glu Phe
        435                 440                 445

Glu Leu Thr Arg Arg Leu Asp His Asp Phe Gly Lys Val Thr Lys Gln
    450                 455                 460

Glu Val Lys Asp Phe Phe Arg Trp Ala Ser Asp His Val Thr Asp Val
465                 470                 475                 480

Ala His Glu Phe Tyr Val Arg Lys Gly Gly Ala Lys Lys Arg Pro Ala
                485                 490                 495

Ser Asn Asp Ala Asp Val Ser Glu Pro Lys Arg Glu Cys Thr Ser Leu
            500                 505                 510

Ala Gln Pro Thr Thr Ser Asp Ala Glu Ala Pro Ala Asp Tyr Ala Asp
        515                 520                 525

Arg Tyr Gln Asn Lys Cys Ser Arg His Val Gly Met Asn Leu Met Leu
    530                 535                 540

Phe Pro Cys Lys Thr Cys Glu Arg Met Asn Gln Ile Ser Asn Val Cys
545                 550                 555                 560

Phe Thr His Gly Gln Arg Asp Cys Gly Glu Cys Phe Pro Gly Met Ser
                565                 570                 575

Glu Ser Gln Pro Val Ser Val Val Lys Lys Lys Thr Tyr Gln Lys Leu
            580                 585                 590

Cys Pro Ile His His Ile Leu Gly Arg Ala Pro Glu Ile Ala Cys Ser
        595                 600                 605

Ala Cys Asp Leu Ala Asn Val Asp Leu Asp Asp Cys Val Ser Glu Gln

610                 615                 620

<210>  7
<211>  624
<212>  PRT
<213>  adeno-associated virus 3B

<400>  7

Met Pro Gly Phe Tyr Glu Ile Val Leu Lys Val Pro Ser Asp Leu Asp
1               5                   10                  15

Glu His Leu Pro Gly Ile Ser Asn Ser Phe Val Asn Trp Val Ala Glu
            20                  25                  30

Lys Glu Trp Glu Leu Pro Pro Asp Ser Asp Met Asp Pro Asn Leu Ile
        35                  40                  45

Glu Gln Ala Pro Leu Thr Val Ala Glu Lys Leu Gln Arg Glu Phe Leu
    50                  55                  60

Val Glu Trp Arg Arg Val Ser Lys Ala Pro Glu Ala Leu Phe Phe Val
65                  70                  75                  80

Gln Phe Glu Lys Gly Glu Thr Tyr Phe His Leu His Val Leu Ile Glu
                85                  90                  95

Thr Ile Gly Val Lys Ser Met Val Val Gly Arg Tyr Val Ser Gln Ile
            100                 105                 110

Lys Glu Lys Leu Val Thr Arg Ile Tyr Arg Gly Val Glu Pro Gln Leu
        115                 120                 125

Pro Asn Trp Phe Ala Val Thr Lys Thr Arg Asn Gly Ala Gly Gly Gly
    130                 135                 140

Asn Lys Val Val Asp Asp Cys Tyr Ile Pro Asn Tyr Leu Leu Pro Lys
145                 150                 155                 160

Thr Gln Pro Glu Leu Gln Trp Ala Trp Thr Asn Met Asp Gln Tyr Leu
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Gln Asn
        195                 200                 205

Pro Asn Ser Asp Ala Pro Val Ile Arg Ser Lys Thr Ser Ala Arg Tyr

210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Ser Lys
            260                 265                 270

Ile Met Ser Leu Thr Lys Thr Ala Pro Asp Tyr Leu Val Gly Ser Asn
        275                 280                 285

Pro Pro Glu Asp Ile Thr Lys Asn Arg Ile Tyr Gln Ile Leu Glu Leu
    290                 295                 300

Asn Gly Tyr Asp Pro Gln Tyr Ala Ala Ser Val Phe Leu Gly Trp Ala
305                 310                 315                 320

Gln Lys Lys Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Glu Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Asp His Asp Phe Gly Lys Val Thr Lys Gln
    450                 455                 460

Glu Val Lys Asp Phe Phe Arg Trp Ala Ser Asp His Val Thr Asp Val
465                 470                 475                 480

Ala His Glu Phe Tyr Val Arg Lys Gly Gly Ala Lys Lys Arg Pro Ala
                485                 490                 495

Ser Asn Asp Ala Asp Val Ser Glu Pro Lys Arg Gln Cys Thr Ser Leu
            500                 505                 510

Ala Gln Pro Thr Thr Ser Asp Ala Glu Ala Pro Ala Asp Tyr Ala Asp
        515                 520                 525

Arg Tyr Gln Asn Lys Cys Ser Arg His Val Gly Met Asn Leu Met Leu
    530                 535                 540

Phe Pro Cys Lys Thr Cys Glu Arg Met Asn Gln Ile Ser Asn Val Cys
545                 550                 555                 560

Phe Thr His Gly Gln Arg Asp Cys Gly Glu Cys Phe Pro Gly Met Ser
                565                 570                 575

Glu Ser Gln Pro Val Ser Val Val Lys Lys Lys Thr Tyr Gln Lys Leu
            580                 585                 590

Cys Pro Ile His His Ile Leu Gly Arg Ala Pro Glu Ile Ala Cys Ser
        595                 600                 605

Ala Cys Asp Leu Ala Asn Val Asp Leu Asp Asp Cys Val Ser Glu Gln
    610                 615                 620

<210>  8
<211>  624
<212>  PRT
<213>  Artificial Sequence

<220>
<223>  baRep protein

<400>  8

Met Pro Gly Phe Tyr Glu Ile Val Leu Lys Val Pro Ser Asp Leu Asp
1               5                   10                  15

Glu His Leu Pro Gly Ile Ser Asn Ser Phe Val Asn Trp Val Ala Glu
            20                  25                  30

Lys Glu Trp Glu Leu Pro Pro Asp Ser Asp Met Asp Pro Asn Leu Ile
        35                  40                  45

Glu Gln Ala Pro Leu Thr Val Ala Glu Lys Leu Gln Arg Glu Phe Leu

50                  55                  60

Val Glu Trp Arg Arg Val Ser Lys Ala Pro Glu Ala Leu Phe Phe Val
65                  70                  75                  80

Gln Phe Glu Lys Gly Glu Thr Tyr Phe His Leu His Val Leu Ile Glu
                85                  90                  95

Thr Ile Gly Val Lys Ser Met Val Val Gly Arg Tyr Val Ser Gln Ile
            100                 105                 110

Lys Glu Lys Leu Val Thr Arg Ile Tyr Arg Gly Val Glu Pro Gln Leu
        115                 120                 125

Pro Asn Trp Phe Ala Val Thr Lys Thr Arg Asn Gly Ala Gly Gly Gly
    130                 135                 140

Asn Lys Val Val Asp Asp Cys Tyr Ile Pro Asn Tyr Leu Leu Pro Lys
145                 150                 155                 160

Thr Gln Pro Glu Leu Gln Trp Ala Trp Thr Asn Met Asp Gln Tyr Leu
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Gln Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Ser Lys
            260                 265                 270

Ile Met Ser Leu Thr Lys Thr Ala Pro Asp Tyr Leu Val Gly Ser Asn
        275                 280                 285

Pro Pro Glu Asp Ile Thr Lys Asn Arg Ile Tyr Gln Ile Leu Glu Leu
    290                 295                 300

Asn Gly Tyr Asp Pro Gln Tyr Ala Ala Ser Val Phe Leu Gly Trp Ala
305                 310                 315                 320

Gln Lys Lys Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Glu Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Glu Phe
        435                 440                 445

Glu Leu Thr Arg Arg Leu Asp His Asp Phe Gly Lys Val Thr Lys Gln
    450                 455                 460

Glu Val Lys Asp Phe Phe Arg Trp Ala Ser Asp His Val Thr Asp Val
465                 470                 475                 480

Ala His Glu Phe Tyr Val Arg Lys Gly Gly Ala Lys Lys Arg Pro Ala
                485                 490                 495

Ser Asn Asp Ala Asp Val Ser Glu Pro Lys Arg Glu Cys Thr Ser Leu
            500                 505                 510

Ala Gln Pro Thr Thr Ser Asp Ala Glu Ala Pro Ala Asp Tyr Ala Asp
        515                 520                 525

Arg Tyr Gln Asn Lys Cys Ser Arg His Val Gly Met Asn Leu Met Leu
    530                 535                 540

Phe Pro Cys Lys Thr Cys Glu Arg Met Asn Gln Ile Ser Asn Val Cys
545                 550                 555                 560

Phe Thr His Gly Gln Arg Asp Cys Gly Glu Cys Phe Pro Gly Met Ser
                565                 570                 575

Glu Ser Gln Pro Val Ser Val Val Lys Lys Lys Thr Tyr Gln Lys Leu
            580                 585                 590

Cys Pro Ile His His Ile Leu Gly Arg Ala Pro Glu Ile Ala Cys Ser
        595                 600                 605

Ala Cys Asp Leu Ala Asn Val Asp Leu Asp Asp Cys Val Ser Glu Gln
    610                 615                 620

<210>  9
<211>  738
<212>  PRT
<213>  Artificial Sequence

<220>
<223>  Spark100 capsid sequence

<400>  9

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asn Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Gln Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Ser Pro Val Lys Thr Ala Pro Gly Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Lys Lys Arg Leu Asn Phe Gly Gln
                165                 170                 175

Thr Gly Asp Ser Glu Ser Val Pro Asp Pro Gln Pro Ile Gly Glu Pro
            180                 185                 190

Pro Ala Ala Pro Ser Gly Val Gly Pro Asn Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ser Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Asn Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Glu Phe Ser Tyr
                405                 410                 415

Asn Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Ser Thr Gly Gly Thr Ala Gly Thr Gln Gln Leu Leu
    450                 455                 460

Phe Ser Gln Ala Gly Pro Asn Asn Met Ser Ala Gln Ala Lys Asn Trp
465                 470                 475                 480

Leu Pro Gly Pro Cys Tyr Arg Gln Gln Arg Val Ser Thr Thr Leu Ser
                485                 490                 495

Gln Asn Asn Asn Ser Asn Phe Ala Trp Thr Gly Ala Thr Lys Tyr His
            500                 505                 510

Leu Asn Gly Arg Asp Ser Leu Val Asn Pro Gly Val Ala Met Ala Thr
        515                 520                 525

His Lys Asp Asp Glu Glu Arg Phe Phe Pro Ser Ser Gly Val Leu Met
    530                 535                 540

Phe Gly Lys Gln Gly Ala Gly Lys Asp Asn Val Asp Tyr Ser Ser Val
545                 550                 555                 560

Met Leu Thr Ser Glu Glu Glu Ile Lys Thr Thr Asn Pro Val Ala Thr
                565                 570                 575

Glu Gln Tyr Gly Val Val Ala Asp Asn Leu Gln Gln Gln Asn Ala Ala
            580                 585                 590

Pro Ile Val Gly Ala Val Asn Ser Gln Gly Ala Leu Pro Gly Met Val
        595                 600                 605

Trp Gln Asn Arg Asp Val Tyr Leu Gln Gly Pro Ile Trp Ala Lys Ile
    610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Asn Gln Ala Lys Leu Ala Ser Phe
            660                 665                 670

Ile Thr Gln Tyr Ser Thr Gly Gln Val Ser Val Glu Ile Glu Trp Glu
        675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Asn Val Asp Phe Ala Val Asn Thr Glu
705                 710                 715                 720

Gly Thr Tyr Ser Glu Pro Arg Pro Ile Gly Thr Arg Tyr Leu Thr Arg
                725                 730                 735

Asn Leu

<210>  10
<211>  738
<212>  PRT
<213>  Artificial Sequence

<220>
<223>  AAVhu37 capsid sequence

<400>  10

Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Ala Ala Lys Thr Ala Pro Gly Lys Lys Arg
    130                 135                 140

Pro Val Glu Pro Ser Pro Gln Arg Ser Pro Asp Ser Ser Thr Gly Ile
145                 150                 155                 160

Gly Lys Lys Gly Gln Gln Pro Ala Lys Lys Arg Leu Asn Phe Gly Gln
                165                 170                 175

Thr Gly Asp Ser Glu Ser Val Pro Asp Pro Gln Pro Ile Gly Glu Pro
            180                 185                 190

Pro Ala Gly Pro Ser Gly Leu Gly Ser Gly Thr Met Ala Ala Gly Gly
        195                 200                 205

Gly Ala Pro Met Ala Asp Asn Asn Glu Gly Ala Asp Gly Val Gly Ser
    210                 215                 220

Ser Ser Gly Asn Trp His Cys Asp Ser Thr Trp Leu Gly Asp Arg Val
225                 230                 235                 240

Ile Thr Thr Ser Thr Arg Thr Trp Ala Leu Pro Thr Tyr Asn Asn His
                245                 250                 255

Leu Tyr Lys Gln Ile Ser Asn Gly Thr Ser Gly Gly Ser Thr Asn Asp
            260                 265                 270

Asn Thr Tyr Phe Gly Tyr Ser Thr Pro Trp Gly Tyr Phe Asp Phe Asn
        275                 280                 285

Arg Phe His Cys His Phe Ser Pro Arg Asp Trp Gln Arg Leu Ile Asn
    290                 295                 300

Asn Asn Trp Gly Phe Arg Pro Lys Arg Leu Ser Phe Lys Leu Phe Asn
305                 310                 315                 320

Ile Gln Val Lys Glu Val Thr Gln Asn Glu Gly Thr Lys Thr Ile Ala
                325                 330                 335

Asn Asn Leu Thr Ser Thr Ile Gln Val Phe Thr Asp Ser Glu Tyr Gln
            340                 345                 350

Leu Pro Tyr Val Leu Gly Ser Ala His Gln Gly Cys Leu Pro Pro Phe
        355                 360                 365

Pro Ala Asp Val Phe Met Ile Pro Gln Tyr Gly Tyr Leu Thr Leu Asn
    370                 375                 380

Asn Gly Ser Gln Ala Val Gly Arg Ser Ser Phe Tyr Cys Leu Glu Tyr
385                 390                 395                 400

Phe Pro Ser Gln Met Leu Arg Thr Gly Asn Asn Phe Glu Phe Ser Tyr
                405                 410                 415

Thr Phe Glu Asp Val Pro Phe His Ser Ser Tyr Ala His Ser Gln Ser
            420                 425                 430

Leu Asp Arg Leu Met Asn Pro Leu Ile Asp Gln Tyr Leu Tyr Tyr Leu
        435                 440                 445

Ser Arg Thr Gln Ser Thr Gly Gly Thr Gln Gly Thr Gln Gln Leu Leu
    450                 455                 460

Phe Ser Gln Ala Gly Pro Ala Asn Met Ser Ala Gln Ala Lys Asn Trp
465                 470                 475                 480

Leu Pro Gly Pro Cys Tyr Arg Gln Gln Arg Val Ser Thr Thr Leu Ser
                485                 490                 495

Gln Asn Asn Asn Ser Asn Phe Ala Trp Thr Gly Ala Thr Lys Tyr His
            500                 505                 510

Leu Asn Gly Arg Asp Ser Leu Val Asn Pro Gly Val Ala Met Ala Thr
        515                 520                 525

His Lys Asp Asp Glu Glu Arg Phe Phe Pro Ser Ser Gly Val Leu Met
    530                 535                 540

Phe Gly Lys Gln Gly Ala Gly Arg Asp Asn Val Asp Tyr Ser Ser Val
545                 550                 555                 560

Met Leu Thr Ser Glu Glu Glu Ile Lys Thr Thr Asn Pro Val Ala Thr
                565                 570                 575

Glu Gln Tyr Gly Val Val Ala Asp Asn Leu Gln Gln Thr Asn Thr Gly
            580                 585                 590

Pro Ile Val Gly Asn Val Asn Ser Gln Gly Ala Leu Pro Gly Met Val
        595                 600                 605

Trp Gln Asn Arg Asp Val Tyr Leu Gln Gly Pro Ile Trp Ala Lys Ile

610                 615                 620

Pro His Thr Asp Gly Asn Phe His Pro Ser Pro Leu Met Gly Gly Phe
625                 630                 635                 640

Gly Leu Lys His Pro Pro Pro Gln Ile Leu Ile Lys Asn Thr Pro Val
                645                 650                 655

Pro Ala Asp Pro Pro Thr Thr Phe Ser Gln Ala Lys Leu Ala Ser Phe
            660                 665                 670

Ile Thr Gln Tyr Ser Thr Gly Gln Val Ser Val Glu Ile Glu Trp Glu
        675                 680                 685

Leu Gln Lys Glu Asn Ser Lys Arg Trp Asn Pro Glu Ile Gln Tyr Thr
    690                 695                 700

Ser Asn Tyr Tyr Lys Ser Thr Asn Val Asp Phe Ala Val Asn Thr Glu
705                 710                 715                 720

Gly Thr Tyr Ser Glu Pro Arg Pro Ile Gly Thr Arg Tyr Leu Thr Arg
                725                 730                 735

Asn Leu